# EUROPEAN PATENT APPLICATION

(11) **EP 4 603 596 A1**
(43) Date of publication of application: **20.08.2025**
(21) Application number: 24305271.9
(22) Date of filing: 16.02.2024
(51) Int. Cl.: C12Q 1/6853

(54) **CHEMICALS, KITS AND METHODS TO QUANTIFY NUCLEIC ACID**

(71) Applicant: Fernandez, Nicolas, 75012 Paris (FR)
(72) Inventor: Fernandez, Nicolas, 75012 Paris (FR)
(74) Representative: Plasseraud IP

(57) **Abstract**

The invention relates to a method for converting a nucleic acid target in a sample into a template oligonucleotide, and optionally for detecting and/or quantifying said nucleic acid target.

The invention further relates to sets of oligonucleotides and kits for use in a method for converting a nucleic acid target in a sample into a template oligonucleotide, and optionally for detecting and/or quantifying said nucleic acid target.

## Description

### FIELD OF THE INVENTION

The present invention relates to methods for detecting and quantifying nucleic acids, in particular by polymerase chain reaction (PCR), as well as reagents for use in such methods.

### BACKGROUND

Digital PCR (dPCR) is a refinement of conventional polymerase chain reaction (PCR) methods in which the sample is distributed into several partitions and where each partition is subjected to a PCR amplification. Specifically, dPCR is used to directly quantify and clonally amplify nucleic acid strands, including DNA or RNA. Hence, dPCR is a very powerful tool to quantify DNA or RNA in a precise, robust and absolute manner. Nevertheless, the precision and the dynamic range of dPCR technics depend on the number of partitions. Mainly for engineering reasons, most existing dPCR equipment uses only some tens of thousands of partitions per sample.

Regarding the dynamic range of precise quantification, due to these limitations, most of the current dPCR systems are limited to about a 2-log range of quantification (relative uncertainty < 5% for 95% confidence) for the concentrations of all the targets of a given assay. In biological samples in which the occurrence of some nucleic acids can vary by many orders of magnitude, an approximation of each of the target concentrations ex-ante is required to choose the proper optimal dilution. Furthermore, in many cases, because some targets are much more concentrated than others in a given sample, it is impossible to quantify them simultaneously in a single assay.

To circumvent this issue, in most instances, the biologist performs several assays with the same sample, optionally at different dilutions of it, in the hope that each target is quantified precisely in at least one chamber or by merging the results of different assays. Nevertheless, this method is costly, requires larger samples, which can be limited for human diagnostics, and does not guarantee adequate precision when values from different chambers are compared. The precision may be impacted, for instance, by pipetting errors, inter-assay variability, or by the sample background effects.

The relative uncertainty of the measurement being dependent on the concentration, even if two targets (of different concentration) fall into the 2-log range, they cannot both be quantified at the optimal uncertainty. Thus, the error on their difference or ratio is usually closer to 10% than to 2%, a level of error that is too high for many applications. For example, a relative uncertainty about 2% is needed in Non-Invasive Prenatal Testing of trisomy. In such cases, the biologist utilizes several chambers in the analysis to average the results or turns to sequencing techniques, which are more precise by design but an order of magnitude more expensive.

There is therefore a need for improved detection and quantification methods of DNA and RNA, in particular by dPCR.

### Summary

In one aspect, the present invention relates to a method for synthesizing a template oligonucleotide from a nucleic acid target in a sample, and optionally for detecting and/or quantifying said nucleic acid target, wherein said method comprises:
a) contacting a sample which contains a target nucleic acid with a nucleic acid polymerase and a first pair of oligonucleotides comprising a first and a second oligonucleotides, wherein:
   (i) the first oligonucleotide comprises a first target-specific sequence and a first bridging sequence, preferably in the 3'-position of the first target-specific sequence, wherein the first target-specific sequence is complementary to a first target region of the target nucleic acid; and
   (ii) the second oligonucleotide comprises a second target-specific sequence and a second bridging sequence, preferably in the 3'-position of the second target-specific sequence, wherein the second target-specific sequence is complementary to a second target region of the target nucleic acid, and wherein the second bridging sequence is complementary to the first bridging sequence,
b) hybridizing the first oligonucleotide to the first target region of the target nucleic acid and hybridizing the second oligonucleotide to the second target region of the target nucleic acid, whereby the first and second bridging sequences hybridize with each other;
c) elongating, by the nucleic acid polymerase, the first and/or second oligonucleotide hybridized by a bridging sequence, thereby inducing the degradation of the target nucleic acid sequence, thus converting the target nucleic acid into the elongated oligonucleotide, providing a template oligonucleotide; and, optionally
d) amplifying the elongated oligonucleotide, preferably by PCR amplification;
e) detecting the presence in the sample of the elongated oligonucleotide or an amplicon amplified therefrom.

In another aspect, the invention relates to a method for detecting and/or quantifying at least two different nucleic acid targets in a sample, wherein said method comprises synthesizing oligonucleotide templates from each nucleic acid target, and optionally amplifying said oligonucleotide templates and detecting the amplicons thereof, according to the method of the invention. In another aspect, the invention relates to a set of oligonucleotides for use in a method for converting and optionally detecting and/or quantifying a target nucleic acid, in particular the method of the invention, comprising a first pair of oligonucleotides, comprising:
(i) a first oligonucleotide comprising a first target-specific sequence and a first bridging sequence, preferably in the 3'-position of the first target-specific sequence, wherein the first target-specific sequence is complementary to a first target region of the target nucleic acid; and
(ii) a second oligonucleotide comprising a second target-specific sequence and a second bridging sequence, preferably in the 3'-position of the second target-specific sequence, wherein the second target-specific sequence is complementary to a second target region of the target nucleic acid and the second bridging sequence is complementary to the first bridging sequence.

In another aspect the invention relates to a kit for use in a method for detecting a target nucleic acid, comprising:
(i) a set of oligonucleotides according to the invention;
and one or more of:
(ii) one or more pair of primers for hybridization to the oligonucleotides of an oligonucleotide pair of the set of oligonucleotides, or to a complement thereof; and
(iii) one or more oligonucleotide probes, wherein each probe is suitable for detecting an oligonucleotide of the set of oligonucleotides;
wherein the kit optionally further comprises at least one of:
(iv) a nucleic acid polymerase;
(v) reagents and buffers for PCR amplification reaction; and
(vi) a notice of instructions or specification.

In another aspect the invention relates to the use of a set of oligonucleotides according to the invention or a kit according to the invention, in a method for detecting and/or quantifying a target nucleic acid, preferably by digital PCR.

### BRIEF DESCRIPTION OF THE DRAWINGS

Fig. 1 shows exemplary embodiments of nucleic acids and reagents used in the invention.
Fig. 2 shows an exemplary embodiment of a method of the invention.
Fig. 3 shows (A) a scheme illustrating a principle for detection of a prior art dPCR method and (B) a diagram showing the relative uncertainty (95% Confidence Interval, "Cl 95%") versus the concentration of a target nucleic acid within the sample for about 20,000 partitions.
Fig. 4 shows a scheme illustrating principle for detection of an exemplary embodiment of the method of the invention.

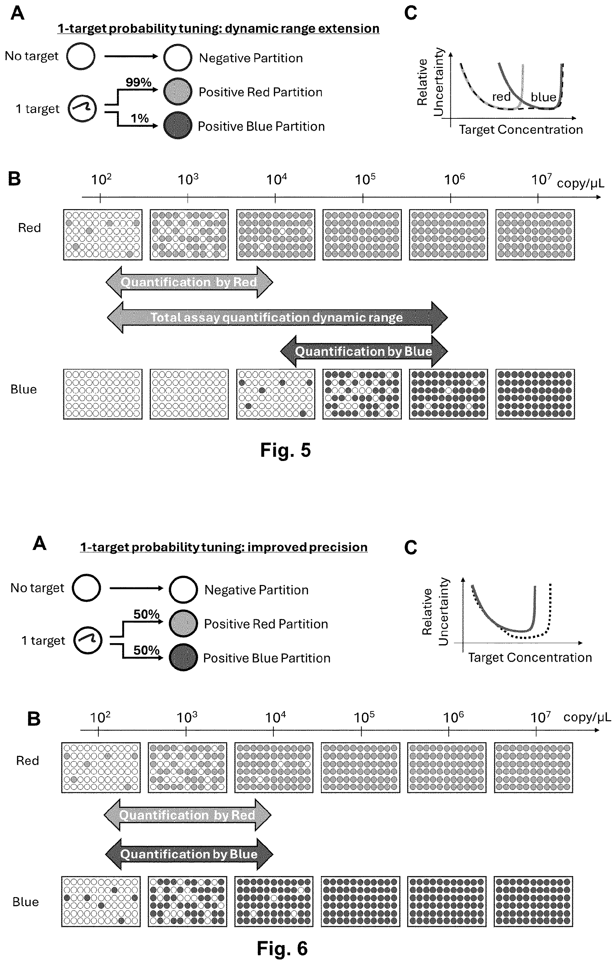

Fig. 5(A) shows a scheme illustrating the principle of probability tuning according to one exemplary embodiment of the method of the invention (dynamic range extension). (B) shows a scheme illustrating signal detection on a sample partitioned in microwell plates versus the concentration of a target nucleic acid within the sample, as obtained by probability tuning in an exemplary embodiment of the method of the invention. (C) shows a schematic diagram illustrating the relative uncertainty versus the concentration of a target nucleic acid within the sample, according to an exemplary embodiment of the invention.
Fig. 6(A) shows a scheme illustrating the principle of probability tuning according to one exemplary embodiment of the method of the invention (improved precision). (B) shows a scheme illustrating signal detection on a sample partitioned in microwell plates versus the concentration of a target nucleic acid within the sample, as obtained by probability tuning in an exemplary embodiment of the method of the invention. (C) shows a schematic diagram illustrating the relative uncertainty versus the concentration of a target nucleic acid within the sample, according to an exemplary embodiment of the invention.
Fig. 7 shows exemplary embodiments of nucleic acids and reagents used in the invention.
Fig. 8 shows an exemplary embodiment of the method of the invention.
Fig. 9 shows exemplary designs of oligonucleotides for use in the present invention.

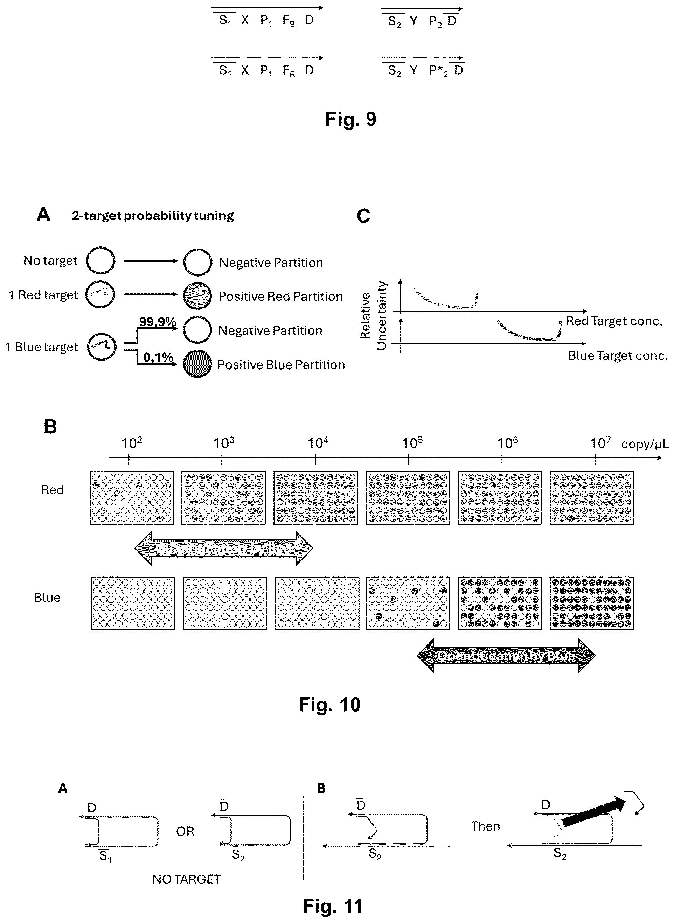

Fig. 10(A) shows a scheme illustrating the principle of probability tuning according to one exemplary embodiment of the method of the invention (two-target probability tuning). (B) shows a scheme illustrating signal detection on a sample partitioned in microwell plates versus the concentration of two target nucleic acids within the sample, as obtained by probability tuning in an exemplary embodiment of the method of the invention. (C) shows a schematic diagram illustrating the relative uncertainty versus the concentration of two target nucleic acids within the sample, according to an exemplary embodiment of the invention.
Fig. 11 shows (A) an exemplary embodiment of a locking oligonucleotide hybridized to a first or second oligonucleotide of the invention (B) an exemplary embodiment of a locking oligonucleotide dehybridization from an oligonucleotide upon hybridization of this oligonucleotide on the target nucleic acid.
Fig. 12 shows different exemplary oligonucleotides that can be used in the method of the invention.
Fig. 13 shows a flowchart listing exemplary steps that may be performed in the method of the invention.

### DETAILED DESCRIPTION OF THE INVENTION

### Definitions

To facilitate comprehension, technical terms are first defined, with additional definitions set forth within the detailed description.

As used herein, the words such as "a," "an," and "the" refer to one or more and encompass the plural unless the context indicates otherwise. Ranges expressed herein may be attributed as "about" one particular value, and/or to "about" another indicated value. The word "about" herein refers to approximately, roughly, in the region of, or around. Use of the word "about" in conjunction with a numerical range, modifies that range by extending the limits to greater or lesser numerical values. Overall, "about" is used to modify a numerical value greater or lesser than the stated value by a variance of approximately 10%. Expression of such a range in another embodiment includes from the one specific value and/or to the other specified value. Similarly, values expressed as approximations, preceded by "about," or a similar word such as "approximately", indicate that the specified value forms another embodiment. Furthermore, the limits of each of the ranges are included with the range.

The word "or" as used herein can refer to any single member of a particular list, as well as any combination of members of that list.

As used herein, the term "amplification of" or "amplifying" an individual templates refers to any kind of nucleic acid amplification method which results in the generation of multiples of the original template. Such operations include, but are not limited to, polymerase chain reaction (PCR), DNA ligase chain reaction (LCR) (see U.S. Pat. Nos. 4,683,195 and 4,683,202); PCR Protocols: A Guide to Methods and Applications (Innis et al., eds, 1990)), RT-PCR, QBeta RNA replicase, and RNA transcription-based (such as TAS) amplification reactions as well as others known to those of skill in the art. Asymmetric PCR, nested PCR, bridge PCR and digital PCR are specific types of amplification reactions well known to those of skill in the art (see for example, but not limited to, Chen et al., Methods Mol Biol., 2011, 231-43; Varley and Mitra, Genome Res., (2008), 1844-50). Amplifying indicates any process of subjecting a nucleic acid to conditions sufficient to permit its amplification. In one embodiment, the nucleic acid amplification reactions disclosed herein can be performed in partitions. Components of an amplification reaction generally include, e.g., primers, template nucleic acids, polymerases, nucleotides, and other components of a similar nature. The term "amplifying" can refer to an exponential increase in template nucleic acid concentration during the amplification reaction, as well as sub-exponential increase, or even linear increase.

As used herein, the term "conversion" indicates synthesis of a nucleic acid from a reference nucleic acid. In preferred embodiments, said conversion is not the synthesis of a complementary strand from a template reference nucleic acid. For instance, the target nucleic acid of the invention may be converted into an oligonucleotide that can be employed as a template in amplification reactions. In some embodiments, the conversion comprises the degradation of the reference nucleic acid, for example by enzymatic hydrolysis.

"Polymerase chain reaction" or "PCR" indicates a sequence of biochemical reactions that achieves amplification of a specific fragment or subsequence of a template nucleic acid. in a template dependent manner. PCR reaction conditions typically comprise three step cycles: (1) a denaturation step, followed by (2) a hybridization step, followed by (3) an elongation step. Standard PCR conditions for the three cycle reactions are characterized by a denaturation (or de hybridization) step at 98° C. for about 1 minute to about 5 minutes in the initial cycle, and about 5 seconds to about 20 seconds for each remaining cycle. A hybridization (or annealing) step at about 45° C. to about 72° C. or at a Tm (melting temperature) at least 1 ° C., 2° C., 3° C., or more, below the melting temperature of the pool of primers, for about 10 seconds to about 1 minute. An elongation or extension step at about 68° C. to about 72° C. for about 15 seconds to about 1 minute, typically about 1 min per kilobase of expected product. The extension step is typically between about 5 minutes and about 10 minutes on the last cycle of the PCR reaction. In some protocols, the hybridization step and the elongation steps are performed simultaneously with duration and conditions that correspond to both phenomena. Generally, a PCR reaction will include between 20 to 30 cycles of the three step PCR conditions described above.

The term "template-dependent", e.g. in "template-dependent manner" or "template-dependent polymerase" typically refers to polynucleotide synthesis of RNA or DNA wherein the sequence of the newly synthesized strand of polynucleotide is complementary to the template, i.e., dictated by the well-known rules of complementary base pairing (see, for example, Watson, J. D. et al., In: Molecular Biology of the Gene, 4th Ed., W. A. Benjamin, Inc., Menlo Park, Calif. (1987)). PCR is well known to those of skill in the art; see, e.g., U.S. Pat. Nos. 4,683,195 and 4,683,202; and PCR Protocols: A Guide to Methods and Applications, Innis et al., eds, 1990.

As used herein, the term "hybridize" or "specifically hybridize" refers to a process where two complementary nucleic acid strands anneal to each other under appropriately stringent conditions. Nucleic acid hybridization techniques are well known in the art. See, e.g., Sambrook, et al., Molecular Cloning: A Laboratory Manual, Second Edition, Cold Spring Harbor Press, Plainview, N.Y.(1989).

As used herein, the term "complementarity" refers to standard Watson-Crick base pairing rules. Complementarity may be partial in which only some of the nucleotide bases of two nucleic acid strands are matched according to the base pairing rules. Two single stranded RNA or DNA molecules are said to be complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%. Alternatively, complementarity exists when an RNA or DNA strand will hybridize under selective hybridization conditions to its complement. Those skilled in the art of nucleic acid technology can determine duplex stability empirically considering a number of variables including, for example, the length of the antisense molecule, base composition and sequence of the antisense molecule, incidence of mismatched base pairs, ionic strength, other hybridization buffer components and conditions.

The term "digital PCR" or "dPCR" refers to a PCR assay performed on portions of a sample to determine the presence/absence, concentration, and/or number of occurrences of a nucleic acid template in the sample (in other words, the copy number of said nucleic acid template), based on how many of the sample portions support amplification of the template. Digital PCR may (or may not) be performed as endpoint PCR. Digital PCR may (or may not) be performed as real-time PCR for each of the partitions. Each partition, e.g. droplet, vessel or well, then provides digital information on the presence or absence of each individual nucleic acid molecule within each partition. When enough partitions are measured using this technique, the digital information can be consolidated to make a statistically relevant measure of starting concentration for the nucleic acid template (analyte) in the sample (see for example, but not limited to Majumdar et al Scientific Reports 2017 7, 9617). In some embodiments, the partitioning comprises generating at least 1 partition, at least 10 partitions, at least 30 partitions that has 0 occurrence of the template molecule. The concept of digital PCR may be extended to other types of analytes, besides nucleic acids. In particular, a signal amplification reaction may be utilized to permit detection of a single copy of a molecule of the analyte in individual droplets, to permit data analysis of droplet signals for other analytes (e.g., using an algorithm based on Poisson statistics). In most applications of dPCR the range of concentrations for which reliable data can be obtained (referred to as the Dynamic Range) are limited to about a 2-log range of quantification (relative uncertainty < 5% for 95% confidence) for all the templates of a given assay. In some embodiments described for the invention herein, the Dynamic Range can be extended to at least 3 logs, in particular from 3 to 10 logs.

In some embodiments, the partitions generated are substantially uniform in shape and/or size. In some embodiments, the partitions generated are non-uniform in shape and/or size. In some embodiments, the partitions generated are substantially uniform in volume. For example, in some embodiments, the microdroplets generated have an average volume between about 0.001 nanoliters (nL) and about 10 nL. In some embodiments, the number of partitions per sample is at least 5,000, at least 10,000, at least 20,000 , at least 50,000 , at least 100,000 , at least 1,000,000, at least 10,000,000.

" Reaction mixture" as used herein refers to the solution constituting the reaction environment. A reaction mixture according to the invention includes any solution known in the art allowing an amplification of nucleic acid molecules. Typical reagents and buffers for a PCR may be selected from magnesium chloride, potassium chloride, dNTP, pH buffer (e.g., tris(hydroxymethyl)aminomethane), additives such as: EDTA, Dimethyl sulfoxide (DMSO), glycerol, formamide, bovine serum albumin (BSA), ammonium sulfate, polyethylene glycol (PEG), gelatin, nonionic detergents (e.g., Tween 20, Triton X-100), N,N,N-trimethylglycine (betaine) and their mixtures. Non-limiting examples of reagents and conditions of PCR reactions are described in Pelt-Verkuil EV, Belkum AV, Hays JP (2008) Principles and Technical Aspects of PCR Amplification. Dordrecht: Springer.

As used herein, the term "partitioning" or "partitioned" refers to separating a sample into several portions ("partitions") separated from each other. Partitions separation can be solid (e.g. microchannel or microwell) or fluid (e.g. emulsion droplet). Methods and compositions for partitioning a sample are described, for example, in published patent applications WO 2010/036352, US 2010/0173394, US 2011/0092373, and US 2011/0092376, each of which is incorporated by reference herein in their entireties.

A "primer" is generally a short single-stranded polynucleotide (e.g. DNA, RNA, or a chimera of DNA and RNA) generally with a free 3'-OH group that binds to a template nucleic acid by hybridizing with a template sequence, and thereafter promotes polymerization of a polynucleotide complementary to the template nucleic acid. Primers can be of a variety of lengths and are often less than 50 nucleotides in length, for example 12-30 nucleotides, in length. In some cases, primers can include one or more modified or non-natural nucleotide bases. The length and sequences of primers for use in PCR can be designed based on principles known to those of skill in the art, see, e.g., Innis et al., supra. In the present application, primers having highly degenerate sequences may be avoided to reduce the probability of mis-hybridization during the PCR annealing reaction.

As used herein, a "primer pair" refers to two polynucleotide sequences that may be the same or different that hybridize to two loci on a template nucleic. Frequently, a primer pair will comprise a forward (upstream) primer and a reverse (downstream) primer that span a region of interest on the template nucleic. In one embodiment, a forward primer comprises the same polynucleotide sequence as the reverse primer.

As used herein, "Tm" alone, refers to melting temperature and is the temperature at which one half of a nucleic acid duplex (e.g., a DNA target nucleic acid) will dissociate to become single-stranded. The melting temperature of a pair of single strand nucleic acids can be influenced, as known in the art, by their sequences, their concentrations as well as the solution properties such as co-solvent, additives, salt concentration.

An oligonucleotide "hybridizes" or "anneals" to another nucleic acid (such that all or only a portion of its sequence are hybridized) under conditions such that non-specific hybridization is minimal at a defined temperature in a physiological buffer (e.g., pH 6-9, 25-150 mM chloride salt). In some cases, a nucleic acid, or portion thereof, hybridizes to a conserved sequence shared among a group of nucleic acids. In some cases, a primer, or portion thereof, can hybridize to a primer binding site, if there are at least about 6, 8, 10, 12, 14, 16, or 18 contiguous complementary nucleotides. Alternatively, a primer, or portion thereof, can hybridize to a primer binding site if there are fewer than 1 or 2 complementarity mismatches over at least about 10, 12, 14, 16, or 18 contiguous complementary nucleotides.

As used herein, a "template" refers to a polynucleotide sequence that comprises the polynucleotide or nucleic acid to be amplified, flanked by one or a pair of primer hybridization sites.

As used herein, a "target nucleic acid" refers to a nucleic acid of interest. The target nucleic acid can include any form such as DNA, RNA, LNA, PNA, locked nucleic acids, mixtures thereof, and hybrids thereof. A target nucleic acid can be composed of any nucleotide sequence comprised by one or more nucleotides or nucleosides, with or without modifications (e.g., capped or terminated nucleotides) or substitutions (e.g., 8-oxo-dG). In some embodiments, a target nucleic acid is between 30 and about 2,000 nucleotides in length, between 30 and about 10,000 nucleotides in length, preferably between 50 and 300 nucleotides in length. In one embodiment, the method of the invention is performed simultaneously on more than one target nucleic acids having different sequences in a sample, for instance, at least 2, at least 10, at least 100 target nucleic acids in a sample. In one embodiment, the method of the invention is performed on a sample, or a partition of a sample, comprising one occurrence of the target nucleic acid, i.e. one molecule of target nucleic acid. In one embodiment, the method of the invention is performed on a sample, or a partition of a sample, comprising more than one occurrence of the target nucleic acid, in particular 2, 3, 4, 5, 6, 7, 8, 9 or 10 or more occurrences of the target nucleic acid. In one embodiment, the sample contains no occurrence of the target nucleic acid.

As used herein, "nucleic acid" includes DNA, RNA, single-stranded, double-stranded, or more highly aggregated hybridization motifs, and any chemical modifications thereof. Nucleic acids can also include natural bases and/or non-natural bases such as, but are not limited to, nitroindole. Modifications can also include 3' and 5' modifications including, but not limited to, capping with a fluorophore or another moiety.

An "oligonucleotide" (or "oligo") refers to a short nucleic acid. It can be completely or partially single stranded and/or double stranded and may contain from a few to about a thousand of nucleotides.

A "polymerase" refers to an enzyme that performs template-directed synthesis of polynucleotides, e.g., DNA and/or RNA. The term encompasses both the full-length polypeptides and a domain that has polymerase activity, including mutant or modified polymerases that retain polymerase activity. DNA polymerases are well-known to those skilled in the art, including but not limited to, DNA polymerases isolated or derived from Thermus aquaticus, Pyrococcus furiosus, Thermococcus litoralis, and Thermotoga maritime, or modified versions thereof. Additional examples of commercially available polymerase enzymes include but are not limited to: Klenow fragment (New England Biolabs^{®} Inc.), Taq DNA polymerase (QIAGEN), 9° N^{™} DNA polymerase (New England Biolabs^{®} Inc.), Deep Vent^{™} DNA polymerase (New England Biolabs^{®} Inc.), Manta DNA polymerase (Enzymatics^{®}), Bst DNA polymerase (New England Biolabs^{®} Inc.), and phi29 DNA polymerase (New England Biolabs^{®} Inc.). Polymerases include both DNA-dependent polymerases and RNA-dependent polymerases such as reverse transcriptase. At least five families of DNA-dependent DNA polymerases are known, although most fall into families A, B and C. There is little or no sequence similarity among the various families. Most family A polymerases are single chain proteins that can contain multiple enzymatic functions including polymerase, e.g. 3' to 5 exonuclease activity, 5 to 3' exonuclease activity or 5' flap endonuclease activity. The exonuclease activities correspond to the hydrolysis of the DNA phosphodiester bonds at the ends of the nucleic acid chain, whereas the 5' flap-endonuclease activity can occur at a position along the nucleic acid chain. Family B polymerases typically have a single catalytic domain with polymerase and 3' to 5' exonuclease activity, as well as accessory factors. Family C polymerases are typically multi-subunit proteins with polymerizing and 3' to 5' exonuclease activity.

5' Flap endonucleases are divalent metal ion-dependent nucleases that act on a nucleic acid duplex (DNA, RNA or DNA/RNA heteroduplex) which has a single-stranded 5' overhang on one of the strands (flap structure). 5' Flap endonucleases catalyse hydrolytic cleavage of the phosphodiester bond at the junction of single- and double-strand. The 5'-flap endonuclease activity can be found in some polymerases such as the Taq polymerase. (Finger LD et al. The wonders of flap endonucleases: structure, function, mechanism and regulation. Subcell Biochem. 2012;62:301-26).

As used herein, a "nucleic acid flap" or flap" refers a single-stranded overhang on one of the strands of a nucleic acid duplex. A flap can be a 5' flap (respectively 3' flap) if the overhang is at the 5' end (respectively 3' end) of a nucleic acid.

Exonucleases are enzymes that catalyse the cleavage of nucleotides, one at a time, from the end of a polynucleotide chain by hydrolysis of phosphodiester bonds, at either the 3' or the 5' end. Depending on the direction of the cleavage, they are classified as 3'-5' exonucleases or 5'-3' exonucleases. Some common examples of exonuclease are Lambda exonuclease, E.coli exonuclease I or Msz exonuclease I. The exonuclease activity can also be found in polymerases. For example, Bst and Taq DNA polymerases exhibit 5'-3' exonuclease activity and T7 and Phi 29 DNA polymerase exhibit 3'-5' exonuclease activity (also described as proof-reading).

The term "sample" refers to a compound, composition, and/or mixture of interest, from any suitable source(s) that contains nucleic acids. Samples may be analysed in their natural state, as collected, and/or in an altered state, for example, following storage, preservation, extraction, lysis, dilution, concentration, purification, filtration, mixing with one or more reagents, partitioning, or any combination thereof, among others. In one embodiment, the sample is a "clinical sample". Clinical samples may include nasopharyngeal wash, blood, plasma, cell-free plasma, huffy coat, saliva, urine, stool, sputum, mucous, wound swab, tissue biopsy, milk, a fluid aspirate, a swab (e.g., a nasopharyngeal swab), and/or tissue from a subject, among others. In some embodiments, the sample may comprise one or several reagents, such as, e.g., an amplification mixture.

As used herein, the term "probe" or "oligonucleotide probe", used interchangeable herein, refers to a structure comprised of a polynucleotide, as defined above, that contains a nucleic acid sequence complementary to a nucleic acid sequence present in the template nucleic acid. The polynucleotide sequences of probes may be composed of DNA, and/or RNA, and/or synthetic nucleotide analogues. Probes are generally of a length compatible with its use in specific detection of all or a portion of a detection sequence of a template nucleic acid, and are usually are in the range of between 8 to 100 nucleotides in length, such as 8 to 75, 10 to 74, 12 to 72, 15 to 60, 15 to 40, 18 to 30, 20 to 40, 21 to 50, 22 to 45, 25 to 40, and so on, more typically in the range of between 18-40, 20-35, 21-30 nucleotides long, and any length between the stated ranges. The typical probe is in the range of between 10-50 nucleotides long, such as 15-45, 18-40, 20-30, 21-28, 22-25 and so on, and any length between the stated ranges.

Probes contemplated herein include probes that include a detectable label. For example, when an "oligonucleotide probe" is to be used in a 5' exonuclease assay, such as the TaqMan^{®} assay, the probe includes at least one fluorescer and at least one quencher which is digested by the 5' exonuclease activity of a polymerase used in the reaction in order to detect any amplified template oligonucleotide sequences. In this context, the oligonucleotide probe will have a sufficient number of phosphodiester linkages adjacent to its 5' end so that the 5' to 3' exonuclease activity employed can efficiently degrade the bound probe to separate the fluorescers and quenchers. Some other examples of probes include the standard probes for PCR such as Molecular beacons or Meltarray probes (see for example, but not limited to Huang et al Proc Natl. Acad. Sci 2022 119 e2110672119).

As used herein, the terms 'tag" and "detectable tag" or "label" and "detectable label" refer to a molecule, or a combination of molecules, capable of detection, including, but not limited to, radioactive isotopes, fluorescers, chemioluminescers, chromophores, enzymes, enzyme substrates, enzyme cofactors, enzyme inhibitors, chromophores, dyes, metal ions, metal sols, ligands (e.g., biotin, avidin, strepavidin or haptens) and the like. The term "fluorescer" refers to a substance or a portion thereof which is capable of exhibiting fluorescence in the detectable range. The term includes combinations of single labeling agents, e.g., a combination of fluorophores that provides a unique detectable signature, e.g., at a particular wavelength or combination of wavelengths. Any method known in the art for conjugating a label to a desired agent may be employed, e.g., using methods described in Hermanson, Bioconjugate Techniques 1996, Academic Press, Inc., San Diego. In some embodiments, the label may be incorporated or attached to a nucleoside, nucleotide, or resulting double-stranded molecule (e.g., SYBR Green) and can include a ligand or dye, for example, a fluorescent dye such as FAM, DABCYL, TAMRA, JOE, ROX, Cy3, Cy5 or Alexa. In one embodiment, the label can include fluorescence resonance energy transfer (FRET). In one embodiment, a FRET label can include two chromophores that are in close proximity to one another (e.g., the donor and acceptor molecule are located on the same or different oligonucleotide strands).

As used herein, the term "synthetic", as applied to "synthetic nucleic acid" or "synthetic oligonucleotide" refers to artificially synthesized polymers of nucleotides, i.e., not extracted from living organisms.

The invention will be further illustrated by reference to the figures and to examples. However, these figures and examples should not be interpreted in any way as limiting the scope of the present invention.

In one aspect, the invention relates to a method for synthesizing an oligonucleotide from a nucleic acid target in a sample, and optionally for detecting and/or quantifying said nucleic acid target, wherein said method comprises:
a) contacting a sample which contains a target nucleic acid with a nucleic acid polymerase and a first pair of oligonucleotides comprising a first and a second oligonucleotides, wherein:
   - the first oligonucleotide comprises a first target-specific sequence and a first bridging sequence, wherein the first target-specific sequence is complementary to a first target region of the target nucleic acid; and
   - the second oligonucleotide comprises a second target-specific sequence and a second bridging sequence, wherein the second target-specific sequence is complementary to a second target region of the target nucleic acid and wherein the second bridging sequence is complementary to the first bridging sequence,
b) hybridizing the first oligonucleotide to the first target region of the target nucleic acid and hybridizing the second oligonucleotide to the second target region of the target nucleic acid, whereby the first and second bridging sequences hybridize with each other; and
c) elongating, by the nucleic acid polymerase, the first and/or second oligonucleotide hybridized by a bridging sequence, wherein the target nucleic acid sequence is degraded.

Said synthesis of the oligonucleotide from a nucleic acid target may also be referred to as a conversion of the nucleic acid target into an oligonucleotide. The nucleic acid target is thus converted.

The method converts the target nucleic acid into the elongated oligonucleotide. In such embodiments, the elongated oligonucleotide produced at step (c) provides a template oligonucleotide, namely an oligonucleotide for use as template in an amplification reaction, e.g. a polymerase chain reaction (PCR). Accordingly, in some embodiments, the method converts the nucleic acid target into a template oligonucleotide.

In some embodiments, the method comprises a prior step of denaturing the target nucleic acid. This is useful in instances where the target nucleic acid is initially present as a double-stranded target nucleic acid, e.g. DNA, and requires at least partial denaturation into single stranded nucleic acid for hybridization of the oligonucleotides of the invention.

In the present invention, a sample comprising a target nucleic acid is contacted with a first oligonucleotide and a second oligonucleotide. The first and/or second oligonucleotides may have any appropriate size provided that they comprise sequences able to hybridize in appropriate conditions, as indicated in the present specification. Design of oligonucleotide sequences fulfilling the hybridization conditions and steps indicated in the present specification may be carried out by the skilled person, using software tools available in the field, such as VNCBI-Blast software.

In some embodiments, the first and/or second oligonucleotides may comprise at least 20 nucleotides, in particular at least about 50 nucleotides. In some embodiments, the first and/or second oligonucleotides may comprise up to about 100, 200 or 1000 nucleotides. For instance, the first and/or second oligonucleotides may comprise from about 20 to about 100 nucleotides.

The sequences of the first and second oligonucleotides are designed such that each of said oligonucleotides is capable to hybridize to a defined region on the target nucleic acid. The first oligonucleotide comprises a first target-specific sequence which is complementary to a first region on the target nucleic acid. The second oligonucleotide comprises a second target-specific sequence which is complementary to a second region on the target nucleic acid. The first and/or second target-specific sequences may have any appropriate size which meets the hybridization requirements of the invention. Preferably, the first and/or second target-specific sequences have from 10 to 40 nucleotides.

The first and second target regions may be any region on the target nucleic acid, in particular such that the secondary structure of the target nucleic acid makes the region accessible for hybridization by the first and/or second oligonucleotides. Preferably, the first and second target regions can be adjacent to each other or can be separated, on the target nucleic acid, by a sequence of up to about 10, up to about 100, up to about 1000 nucleotides. In some embodiments, the second target region is in the 5'-position of the first target region. In other embodiments, the second target region is in the 3'-position of the first target region. The first and second target-specific region have a melting temperature (Tm), with their respective target region, corresponding to about, or close to about what is recommended for standard PCR primers, e.g. from about 45°C to about 75°C.

The first and second oligonucleotide further comprise, respectively, a first bridging sequence and a second bridging sequence. The first and second bridging sequences are complementary. The first and second bridging sequences are capable to hybridize to each other in appropriate conditions, as described in the present specification. Due to the hybridization of the first and second binding sequences, the first and second oligonucleotides are hybridized with each other.

In some embodiments, the first bridging sequence is located in a 3'-position relative to the first target-specific sequence. In some embodiment, the second bridging sequence is located in a 3'-position relative to the second target-specific sequence. Preferably, the first bridging sequence is located in a 3'-position relative to the first target-specific sequence and the second bridging sequence is located in a 3'-position relative to the second target-specific sequence.

In specific embodiments, the first bridging sequence and the second bridging sequences are located at the 3'-end of the first and second oligonucleotides, respectively. In other specific embodiments, only one of the first bridging sequence and the second bridging sequences are located at the 3'-end of the first or the second oligonucleotides.

The first and/or second bridging sequences may have any sequence and/or any size as long as they are complementary and fulfil the requirements as indicated in the present specification. In particular, the first bridging sequence and second bridging sequence may be designed such as to have a duplex melting temperature (Tm) lower than the duplex Tm of the first target-specific sequence with the first target region of the target nucleic acid, and/or lower than the hybridization Tm of the second target-specific sequence with the second target region of the target nucleic acid. In some particular embodiments, the first and second bridging sequences are not identical or complementary to any other sequence in the system. In these embodiments, these sequences are absent or rarely present in biological samples in particular naturally occurring biological samples.

In step (c), nucleic acid polymerase activity generally requires the formation of a stable duplex of the first and second oligonucleotides. In particular, the duplex must have a sufficient long half-life for the polymerase to be active on the duplex elongation. In some embodiments, the first oligonucleotide and the second oligonucleotide do not hybridize with each other in free solution, e.g. at step (a), or do not hybridize sufficiently stably in free solution, for the nucleic acid polymerase-mediated elongation to occur. In particular, hybridization of the first oligonucleotide and second oligonucleotide and the resulting elongation, is brought about by the hybridization of the first oligonucleotide and second oligonucleotide to the target nucleic acid. This property can be obtained through the design of the oligonucleotides, the solution temperature or chemical composition and the oligonucleotide concentrations. Said hybridization of the first and second bridging sequences with each other preferably occurs due to a local concentration effect. In effect, i.e., said sequences hybridize only when they are maintained in sufficient proximity to each other. This occurs, for instance, when the first and second bridging sequences are brought into proximity by the binding of the first oligonucleotide and second oligonucleotide to the target nucleic acid, through the binding of the first target-specific sequence to the first nucleic acid region, and the binding of the second target-specific sequence to the second nucleic acid region, respectively. Hence, in some embodiments, the first oligonucleotide and second oligonucleotide hybridize with each other upon hybridization of the first oligonucleotide and second oligonucleotide to the target nucleic acid.

The sample may be contacted at step (a) with a nucleic acid polymerase. The nucleic acid polymerase is typically a template-dependent nucleic acid polymerase. Template-dependent nucleic acid polymerases are capable of elongating a nucleic acid hybridized to a template and, therefore, of amplifying said nucleic acid. Preferably, the nucleic acid polymerase has 5'-flap endonuclease activity. Preferably, nucleic acid polymerase has 5'->3' exonuclease activity. Most preferably, it has 5'->3' exonuclease activity and 5'-flap endonuclease activity. Preferably, the polymerase is a thermostable polymerase that can maintain its activity despite of thermic treatment of the sample such as double strand denaturation or PCR cycles. In particular, the polymerase is from family A, preferably from type I. More preferably, the polymerase is selected from DNA polymerase I from E. coli or its natural or synthetic mutants, and Taq polymerase or its natural or synthetic mutants. In most preferred embodiments, the nucleic acid polymerase is selected from Taq polymerase and its variants.

Step (b) of the method comprises hybridizing the first oligonucleotide to the first target region of the target nucleic acid and hybridizing the second oligonucleotide to the second target region of the target nucleic acid, whereby the first and second bridging sequences hybridize with each other. The method is thus carried out in conditions such that said hybridization occurs, in particular under conditions of concentration of the first and second oligonucleotides. The sample is also set under conditions of temperature such that hybridization of the first and second oligonucleotides to the target nucleic acid occurs, in particular above 40°C, above 50°C, above 60°C, above 70°C; in particular between 50°C and 70°C.

The method further comprises a step (c) which may comprise elongating the first and/or second oligonucleotide hybridized by a bridging sequence. Said elongation is preferably carried out by the nucleic acid polymerase, via a sequential addition of nucleotides to the end, preferably the 3'-end of one of the first and/or second oligonucleotide, based on the hybridized template consisting of the other of the first and/or second oligonucleotide.

In a preferred embodiment, elongation of the first and/or second oligonucleotide induces degradation of the target nucleic acid. In particular, the target nucleic acid is degraded at step (c) by hydrolysis of the target nucleic acid catalyzed by a 5'-flap endonuclease activity and a 5'->3' exonuclease activity. Upon elongation, the first and/or second oligonucleotides indeed form a flap structure at the junction with the target nucleic acid, i.e. at the boundary of the duplex formed by hybridization of the first and/or second oligonucleotide with the respective complementary regions of the target nucleic acid.

The target nucleic acid may be hydrolyzed, at the level of this flap structure, by a 5'-flap endonuclease activity. The internal break in the target nucleic acid caused by the 5'-flap endonuclease activity may be followed by an external digestion of the free 5'-end of the target nucleic acid, by a 5'->3' exonuclease.

Preferably, the 5'-flap endonuclease activity and the 5'-3' exonuclease activities are expressed by the same enzyme. Preferably, the polymerase activity, the 5'-flap endonuclease activity and the 5'->3' exonuclease activities are expressed by the same enzyme. That is, the nucleic acid polymerase used in the method preferably has 5'-flap endonuclease activity and 5'->3' exonuclease activity. In such embodiments, the target nucleic acid sequence is degraded at step (c) by the 5'-flap endonuclease activity and 5'->3' exonuclease activity of the nucleic acid polymerase.

It is particularly advantageous that the elongation of the first and/or second oligonucleotide be accompanied by a degradation of the target nucleic acid, as this eliminates the target nucleic acid from the sample. As a consequence, one occurrence of the target nucleic acid is converted into one occurrence of the elongated first oligonucleotide and/or one occurrence of the elongated second oligonucleotide. This provides a controlled conversion of the target nucleic acid in one ortwo template oligonucleotides, available for further amplification, and ensures that the quantity of template oligonucleotides can be directly connected to the quantity of target nucleic acids in the sample. Degradation of the target nucleic acid upon conversion into template oligonucleotide(s) also prevents the hybridization of another pair of first and second oligonucleotides on the same target nucleic acid that could lead to the creation of another set of template oligonucleotides and errors in the quantification of the target nucleic acid. Degradation of the target nucleic acid thus provides the opportunity to control the number of oligonucleotide templates in the reaction and, therefore, the type and amplitude of the signal obtained from said templates. Where the method is designed so as to provide several types of detection signals, e.g. several types of fluorescent signals and/or an absence of signal, for a same target nucleic acid, e.g. by using several competitive pairs of oligonucleotides, it is advantageous that one occurrence of target nucleic acid give rise to the detection of a single type of signal, e.g. a single fluorescent signal or an absence of signal, and does not give rise to the detection of mixed signals. In such an embodiment, the detection of mixed signal will reflect the presence of several, i.e., at least two target nucleic acid occurrences within the sample.

In some embodiments, the method of the invention can be repeated for several cycles of conversions such as to optimize the percentage of converted nucleic acid. In particular, at least 90%, preferably at least 95%, more preferably at least 98%, most preferably at least 99% or 100% nucleic acid within the sample or the partitions of a partitioned sample, can be converted. During each cycle of conversion, the sample is preferably put in denaturing conditions such as to dehybridize the duplexes formed. In some embodiments, the method thus comprises repeating cycles of steps (a) to (c). Preferably, the method further comprises a denaturation step between each cycle of steps (a) to (c).

In some embodiments, the nucleic acid target is converted at a ratio of about 1 elongated oligonucleotide synthesized per occurrence of nucleic acid target present in the sample, corresponding to the elongation of the first oligonucleotide or the second oligonucleotide into an elongated oligonucleotide. In some embodiments, the nucleic acid target is converted at a ratio of about 2 oligonucleotides synthesized per occurrence of nucleic acid target present in the sample, corresponding to the elongation of the first oligonucleotide and the second oligonucleotide into elongated oligonucleotides.

In some embodiments, the nucleic acid target is converted at a ratio of about 1 elongated oligonucleotide synthesized per occurrence of first and/or second oligonucleotides contacted with the sample.

In some embodiments, the method further comprises a step d) of amplifying the elongated oligonucleotide, in particular the template oligonucleotide. In some embodiments, said first oligonucleotide comprises a first priming sequence and the second oligonucleotide comprises a second priming sequence, wherein at step (c), the elongated oligonucleotide comprises one of the first and second priming sequences and a complement of the other of the first and second priming sequences. In some embodiments, step (d) comprises amplifying the elongated oligonucleotide by contacting the elongated oligonucleotide with a pair of primers capable of priming amplification of the elongated oligonucleotide by hybridization to the first and second priming sequences or their complement. Said pair of primers therefore comprises sequences complementary to the elongated oligonucleotide and can hybridize to the elongated oligonucleotide and prime amplification of the elongated oligonucleotide.

In one embodiment, the first and/or priming sequences have a melting temperature with the corresponding primers, that is higher than the melting temperature of the bridging sequence. Such design simplifies the possible side-reactions during the PCR cycles.

Amplification of the elongated oligonucleotides is preferably exponential. It is preferably carried out by PCR. Hence, the amplification reaction is preferably guided by the presence of one or several primer pairs. The reaction conditions are typically those of a PCR, i.e. application of cycles of temperature applications, for predefined time periods. Typical conditions for the PCR cycle are those used in digital PCR, i.e. as described in Huggett, Jim F., et al. "The Digital MIQE Guidelines: Minimum Information for Publication of Quantitative Digital PCR Experiments." Clinical chemistry 59.6 (2013): 892-902. PCR enables exponential amplification of the elongated oligonucleotides, if such oligonucleotides comprise a priming sequence for which a primer is introduced in the amplification reaction mixture during the method. The design of the oligonucleotide is such that, if the elongated oligonucleotide comprises a priming sequence for which no complementary primer is introduced during the method, such as sequence P1* or P2* as illustrated in the figures, then such oligonucleotide is not amplified exponentially during the method. Only a linear amplification is possible, which does not interfere with the exponential amplification simultaneously carried out by the pairs of primers introduced in the reaction.

In some embodiments, the primers and probes used to hybridize to the amplicons, do not hybridize to the target nucleic acid in the reaction conditions of the method of the invention. Accordingly, the amplicons amplified from the elongated oligonucleotide preferably do not comprise a sequence comprised in the target nucleic acid and/or a sequence complementary to the target nucleic acid, in particular sequences of at least 6, 8, 10, 12, 14, 16, 18 or 20 consecutive nucleotides comprised within the target nucleic acid. In particular embodiments, the sequences of the amplicons are absent or rarely present in biological samples, particularly naturally occurring biological samples, in particular sequences of at least 6, 8, 10, 12, 14, 16, 18 or 20 consecutive nucleotides comprised within the target nucleic acid. This applies, in particular, to the priming sequences and/or detection sequences present within the amplicons. Using amplicons without significant sequences from the target nucleic acid/from natural origin allows using a standard set of primers and detection probes for any type of biological sample and target nucleic acid, providing considerable advantages in terms of cost-efficiency and practicability of the method.

In some embodiments, the method further comprises a step (e) of detecting the presence in the sample of the elongated oligonucleotide or an amplicon amplified therefrom. In particular, the first and/or second oligonucleotide may comprise a detection sequence such that the elongated oligonucleotide comprises the detection sequence or a complement thereof. In some embodiments, step (e) comprises hybridizing the elongated oligonucleotide or an amplicon amplified therefrom with a detectable oligonucleotide probe comprising a sequence complementary to the detection sequence or a complement thereof. The detectable oligonucleotide probe may comprise a detectable moiety such as a fluorescent probe. In some embodiments, said fluorescent probe is not detectable, e.g. quenched, when the detectable oligonucleotide probe is not hybridized to the elongated oligonucleotide or an amplicon amplified therefrom, for instance due to fluorescence quenching by a second fluorescent probe. In such embodiments, hybridization of the detectable probe to the elongated oligonucleotide or an amplicon thereof, may lead to exonuclease-mediated degradation of the detectable probe, such as to make the probe detectable by removing fluorescence quenching. Examples of suitable oligonucleotide probes are TaqMan^{®} probes.

The detection sequence may be present on the first oligonucleotide, on the second oligonucleotide, on both oligonucleotides or on neither of them. Where a detection sequence is present on both oligonucleotides, a same or different detection sequence may be comprised in both oligonucleotides. Using different detection sequences and, accordingly, different distinctly detectable probes (e.g. fluorescent probes of different colors), in the first and second oligonucleotides of a same oligonucleotide pair, increases the quantitation by providing two distinct signals from a single converted oligonucleotide, instead of one. Where the detection sequence is present on only one oligonucleotide from the first and second oligonucleotides, this means that the sample is not contacted during the method, with a detectable oligonucleotide probe, in particular a fluorescently detectable oligonucleotide probe capable of hybridizing with the oligonucleotide which does not comprise a detecting sequence.

In some embodiments, the first and/or second oligonucleotides comprise one or two spacer regions non-complementary with the target nucleic acid, wherein said regions are adjacent to the first and/or second target-specific regions, respectively, preferably in the 3'-position relative to the first and/or second target-specific regions, respectively, such that said spacer region does not hybridize with the target nucleic acid at step (b). In some embodiments, the spacer regions are present in a 5'-position relative to the first and/or second target-specific regions. In some embodiments, the first and/or second target-specific regions are flanked by spacer regions in 5' and 3'-positions relative to the first and/or second target-specific regions. During elongation of the first and/or second oligonucleotides at step (c), the polymerase elongates up to and including a said spacer region before arriving at the flap structure, formed at the boundary of the target-specific region and a spacer region. The sequence of the spacer regions contributes to a fine tuning of the flap surroundings. In some embodiments, a spacer region can have a degenerate sequence.

The different sequences or regions within the first and/or second oligonucleotides may be present in various orders so long as hybridization of the first and second oligonucleotides is possible with the target nucleic acid and induces the hybridization of the first and second bridging sequences and, consequently, elongation of the first and/or second oligonucleotide sequences. For instance, each of the detection sequence and/or the first and/or second priming sequences may be positioned in the 3'-position or in the 5'-position of the first and/or second target specific sequences, respectively in the first and/or second oligonucleotides. In a particular embodiment, illustrated in Fig. 9, the first oligonucleotide comprises, in 5' to 3' orientation, the first target specific sequence, a first spacer sequence, a first tag sequence and the first bridging sequence and/or the second oligonucleotide comprises, in a 5' to 3' orientation, the second target specific sequence, a second spacer sequence and the second bridging sequence. Such orders of functional regions and sequences in the oligonucleotides of the invention are only illustrative and other orders may be readily designed and used by the skilled person.

In some embodiments, before being contacted with the sample, said first oligonucleotide and/or second oligonucleotides are locked by hybridization to a first and/or second locking oligonucleotide, respectively, such that they do not hybridize with each other in the absence of the target nucleic acid, wherein the first locking oligonucleotide comprises a first locking sequence complementary to a part of the first target-specific sequence and a second locking sequence complementary to at least a part of the first bridging sequence, such as to hybridize the first oligonucleotide through the first and second locking sequences, and wherein the second locking oligonucleotide comprises a third locking sequence complementary to a part of the second target-specific sequence and a fourth locking sequence complementary to at least a part of the second bridging sequence. Upon contact of the first and second oligonucleotides with the target nucleic acid within the sample, the first and second oligonucleotides hybridize to the target nucleic acid, whereby the first and second oligonucleotides dehybridize from the first and second locking oligonucleotides, respectively. When bound to the first or second oligonucleotides, said first or second locking oligonucleotides adopt a staple shape, which blocks the oligonucleotides from binding with each other. The staple-shaped lock is unbound only upon hybridization by the target nucleic acid. This prevents the first or second oligonucleotides from forming a duplex before being contacted with the target nucleic acid and guarantees that every elongation from the duplex formed by the first or second oligonucleotides results from a simultaneous hybridization of said first and second oligonucleotides to the target nucleic acid. On Fig. 11, examples of locking oligonucleotide are provided. In the figure, the locking oligonucleotide is partially complementary, on 5' end, with the bridging sequence of the oligonucleotide and, on the 3' end, with target-specific sequence S1 or S2, in such a way that it does prevent the hybridization of the bridging sequence with another polynucleotide of the solution and cannot be used as a priming site for extension, (b) Because the locking oligonucleotide does not block the whole target-specific sequence, the oligonucleotide can still hybridize on the target and the hybridization competition between the locking oligonucleotide and the target nucleic acid, which has a longer complementary sequence, leads to the local dehybridization of the locking oligonucleotide. By design, the hybridization of the locking oligonucleotide on the bridging sequence is not sufficient to maintain, alone, the link with the oligonucleotide. Thus, the locking oligonucleotide dehybridizes from the bridging sequence that is then available for annealing for example with another oligonucleotide from the same pair. The locking oligonucleotide, thus, helps to prevent the annealing of the two oligonucleotides of a pair in absence of the target.

In some embodiments, the sample is contacted, at step (a), with at least one second pair of oligonucleotides. Accordingly, in some embodiments, the sample is contacted, at step (a), with at least two pairs of oligonucleotides.

The second pair of oligonucleotides preferably comprises a third oligonucleotide and a fourth oligonucleotide, wherein:
(i) the third oligonucleotide comprises a third target-specific sequence and a third bridging sequence, preferably located in the 3'-position relative to the third target-specific sequence, wherein the third target-specific sequence is complementary to the first target region of the target nucleic acid; and
(ii) the fourth oligonucleotide comprises a fourth target-specific sequence and a fourth bridging sequence, preferably located in the 3'-position relative to the fourth target-specific sequence, wherein the fourth target-specific sequence is complementary to the second target region of the target nucleic acid, wherein the fourth bridging sequence is complementary to the third bridging sequence.

Preferably, the fourth bridging sequence is complementary to the first bridging sequence and/or the third bridging sequence is complementary to the second bridging sequences.

In some embodiments, the fourth bridging sequence is complementary to the first bridging sequence. Consequently, the fourth bridging sequence competes with the second bridging sequence for hybridization to the first oligonucleotide sequence. Thus, the fourth oligonucleotide competes with the second oligonucleotide for hybridization to the first oligonucleotide.

In some embodiments, the third bridging sequence is complementary to the second bridging sequence. Consequently, the third bridging sequence competes with the first bridging sequence for hybridization to the second oligonucleotide sequence. Thus, the third oligonucleotide competes with the first oligonucleotide for hybridization to the second oligonucleotide.

In some embodiments, the third bridging sequence is identical to the first bridging sequence. In some embodiments, the fourth bridging sequence is identical to the second bridging sequence. In some embodiments, the third and fourth bridging sequence are respectively identical to the first and second bridging sequences.

In some embodiments, at step (b), the third oligonucleotide competes with the first target-specific sequence for hybridization to the target nucleic acid and the fourth oligonucleotide competes with the second target-specific sequence for hybridization to the target nucleic acid. As a consequence, the target nucleic acid may be hybridized to the first and second oligonucleotides, or the target nucleic acid may be hybridized to the third and fourth oligonucleotides. In particular:
- if the target nucleic acid is hybridized to the first and second oligonucleotides, step (c) comprises elongating, by the nucleic acid polymerase, the first and/or second oligonucleotides hybridized by a bridging sequence, thereby inducing the degradation of the target nucleic acid sequence, whereby the target nucleic acid is converted into an oligonucleotide elongated from the first and/or second oligonucleotides;
Alternatively, if the target nucleic acid is hybridized to the third and fourth oligonucleotides, step (c) comprises elongating, by the nucleic acid polymerase, the third and/or fourth oligonucleotides hybridized by a bridging sequence, thereby inducing the degradation of the target nucleic acid sequence, whereby the target nucleic acid is converted into an oligonucleotide elongated from the third and/or fourth oligonucleotides.

Conversion of a target nucleic acid into an oligonucleotide template originating from the first or second pair of oligonucleotides is a stochastic process, governed only by the hazard in view of the imposed reaction conditions (such as oligonucleotide concentrations, designs, temperatures, buffer and reagents).

In some embodiments, the method comprises the use of a second oligonucleotide pair which is not exponentially amplified during the performance of the method. Said oligonucleotide pair may be referred to as "sterile" oligonucleotide pair, which does not give rise to a detected signal. This occurs, preferably, through the absence, during the method, of at least one primer of the primer pair necessary for amplification of the second oligonucleotide pair.

Accordingly, in some embodiments, the method does not comprise exponentially amplifying all occurrences of elongated oligonucleotides synthesized in the method, i.e. all occurrences of elongated oligonucleotide templates converted by conversion steps (a) to (c). In some embodiments, step (d) comprises exponentially amplifying an elongated oligonucleotide synthesized from the first pair of oligonucleotides and does not comprise exponentially amplifying elongated oligonucleotide synthesized from said second pair of oligonucleotides. Accordingly, in some embodiments, the method does not include a step of exponential amplification of oligonucleotides elongated from said second pair of oligonucleotides. In particular, the method does not include a step of contacting the sample with a primer pair specific for said second pair of oligonucleotides or oligonucleotides elongated therefrom at step (b), in particular a primer pair capable of amplifying, in particular exponentially amplifying, said second pair of oligonucleotides or oligonucleotides elongated therefrom. As a consequence, oligonucleotides converted from the third and/or fourth oligonucleotides are not amplified in the method and, consequently, are not detected in the method. Preferably, the method does not comprise contacting at least one of the third and/or fourth elongated oligonucleotide with a complementary oligonucleotide capable of priming an amplification reaction from said elongated oligonucleotide, preferably exponential amplification, most preferably by PCR. The absence of exponential amplification of the elongated oligonucleotides from the second pair of oligonucleotides also helps to reduce the competition between multiple-template amplifications within the same volume or partition.

In some embodiments, only one of the third and fourth oligonucleotides is not contacted with a complementary primer during the performance of the method. The other of the third and fourth oligonucleotide may be contacted with a complementary primer. Typically, where one of the third and fourth oligonucleotide is identical to one of the first and second oligonucleotide, it is complementary to one of the primers introduced in the sample to amplify the first oligonucleotide pair. This is however not sufficient to exponentially amplify the second oligonucleotide pair, in the absence of a second primer specific for the second oligonucleotide pair. Non-exponential amplification which may occur on the sterile oligonucleotide does not influence the detection of the first oligonucleotide pair.

Using a sterile, i.e. non- exponentially amplified, oligonucleotide pair in the reaction mixture allows tuning the probability of detecting a signal corresponding to a target nucleic acid, in a given sample, for example in a partition of a partitioned sample. The proportion of oligonucleotides converted from the "sterile" oligonucleotide pair, i.e. non-exponentially amplified oligonucleotide pair correspond to the second oligonucleotide pair, in comparison to oligonucleotides converted from the "fertile" oligonucleotide pair, i.e. first oligonucleotide pair may be adjusted, e.g. in terms of oligonucleotide concentration within the reaction mixture, such that a given target nucleic acid gives rise to a predefined probability of generating a signal after amplification.

In some embodiments, the first and second oligonucleotide pairs have one oligonucleotide in common. Hence, one of the third and fourth oligonucleotides may be identical to one of the first and second oligonucleotides. In particular, the third oligonucleotide may be identical to the first oligonucleotide, or the fourth oligonucleotide may be identical to the third oligonucleotide.

Accordingly, in some embodiments, contacting the sample with a second oligonucleotide pair amounts to contacting it with one additional oligonucleotide only, i.e., an auxiliary oligonucleotide, in addition to the first oligonucleotide pair.

Accordingly, in some embodiments of the method, the sample is contacted at step (a), with at least one auxiliary oligonucleotide, wherein:
(iii) the auxiliary oligonucleotide comprises an auxiliary target-specific sequence and an auxiliary bridging sequence, preferably located in the 3'-position of the auxiliary target-specific sequence, wherein the auxiliary target-specific sequence is complementary to the first target region of the target nucleic acid,
wherein the auxiliary bridging sequence is complementary to the second bridging sequence,
wherein at step (b), the auxiliary oligonucleotide competes with the first oligonucleotide for hybridization to the target nucleic acid, wherein:
   - if the target nucleic acid is hybridized to the first and second oligonucleotides, step (c) comprises elongating, by the nucleic acid polymerase, the first and/or second oligonucleotides hybridized by a bridging sequence, thereby inducing the degradation of the target nucleic acid sequence, whereby the target nucleic acid is converted into an oligonucleotide elongated from the first and/or second oligonucleotides; or
   - if the target nucleic acid is hybridized to the auxiliary and second oligonucleotides, step (c) comprises elongating, by the nucleic acid polymerase, the auxiliary and/or second oligonucleotides hybridized by a bridging sequence, thereby inducing the degradation of the target nucleic acid sequence, whereby the target nucleic acid is converted into an oligonucleotide elongated from the auxiliary and/or second oligonucleotides.

In other embodiments of the method, the sample is contacted at step (a), with at least one auxiliary oligonucleotide, wherein:
(iii) the auxiliary oligonucleotide comprises an auxiliary target-specific sequence and an auxiliary bridging sequence, preferably located in the 3'-position of the auxiliary target-specific sequence, wherein the auxiliary target-specific sequence is complementary to the second target region of the target nucleic acid,
wherein the auxiliary bridging sequence is complementary to the first bridging sequence,
wherein at step (b), the auxiliary oligonucleotide competes with the second oligonucleotide for hybridization to the target nucleic acid, wherein:
   - if the target nucleic acid is hybridized to the first and second oligonucleotides, step (c) comprises elongating, by the nucleic acid polymerase, the first and/or second oligonucleotides hybridized by a bridging sequence, thereby inducing the degradation of the target nucleic acid sequence, whereby the target nucleic acid is converted into an oligonucleotide elongated from the first and/or second oligonucleotides; or
   - if the target nucleic acid is hybridized to the first and auxiliary oligonucleotides, step (c) comprises elongating, by the nucleic acid polymerase, the first and/or auxiliary oligonucleotides hybridized by a bridging sequence, thereby inducing the degradation of the target nucleic acid sequence, whereby the target nucleic acid is converted into an oligonucleotide elongated from the first and/or auxiliary oligonucleotides.
The features described in relation to the third and/or fourth oligonucleotide are applicable to the auxiliary oligonucleotide. In particular, the auxiliary oligonucleotide is preferably a sterile oligonucleotide, i.e. it can form an oligonucleotide pair with the first or second oligonucleotide, which pair may form a duplex and may be elongated but is not amplified. Preferably, the method does not include contacting the sample with a primer capable of hybridizing to the auxiliary oligonucleotide and, particularly, capable of amplifying oligonucleotides templates elongated from an oligonucleotide pair including the auxiliary oligonucleotide.

In some embodiments, the method comprises a step of amplifying an oligonucleotide elongated from the second pair of oligonucleotides. In such case, the second pair of oligonucleotides is capable of giving rise to a signal (i.e. is "fertile"), similarly to the first oligonucleotide pair. In particular, the third oligonucleotide comprises a third priming sequence and the fourth oligonucleotide comprises a fourth priming sequence, wherein at step (c), an oligonucleotide elongated from the third and/or fourth oligonucleotides, i.e. from the second oligonucleotide pair, comprises one of the third and fourth priming sequences and a complement of the other of the third and fourth priming sequences. In some embodiments, step (d) comprises amplifying the elongated oligonucleotide by contacting the elongated oligonucleotide with a pair of primers capable of priming amplification of the elongated oligonucleotide by hybridization to the third and fourth priming sequences or their complement. Said pair of primers therefore has sequences complementary to the elongated oligonucleotide and can hybridize to the elongated oligonucleotide and prime amplification of the elongated oligonucleotide. In a preferred embodiment, at least one of the third and fourth priming sequences is not identical to the first and second priming sequences in order to allow the control the competition between the amplification of elongated oligonucleotides by the primer concentrations.

In some embodiments, the method further comprises a step (e) of detecting the presence in the sample of the oligonucleotide elongated from the second oligonucleotide pair, or an amplicon amplified therefrom. In particular, the third and/or fourth oligonucleotide may comprise a detection sequence such that the elongated oligonucleotide comprises the detection sequence or a complement thereof. In some embodiments, step (e) comprises hybridizing the elongated oligonucleotide or an amplicon amplified therefrom with a detectable oligonucleotide probe comprising a sequence complementary to the detection sequence or a complement thereof. The detection sequence comprised in the third and/or fourth oligonucleotide is preferably different from the detection sequence comprised in the first and/or second oligonucleotide such as to hybridize distinct oligonucleotide probes and generate distinct signals at step (e). The oligonucleotide probes which are contacted with the sample to hybridize to the oligonucleotide elongated from the second oligonucleotide pair, or an amplicon amplified therefrom, generate a distinct signal from the oligonucleotide probes which are contacted with the sample to hybridize to the oligonucleotide elongated from the first oligonucleotide pair, or an amplicon amplified therefrom. In particular, different oligonucleotide probes with different distinctly detectable fluorescent tags may be used to generate a different signal for each oligonucleotide pair. Examples of suitable oligonucleotide probes are TaqMan^{®} probes.

The design of the third and/or fourth oligonucleotides may be similar to the design of the first and/or second oligonucleotides, respectively. In particular, the third and/or fourth oligonucleotide may have a sequence identical to the first and/or second oligonucleotide, respectively, in which the detection sequence, if present, is replaced by a different detection sequence, capable of hybridizing different oligonucleotide probes. The third and/or fourth oligonucleotide may also further comprise a priming sequence which may be different from the priming sequence of the first and/or second oligonucleotide, respectively. In some embodiments, as described previously, one of the first and second oligonucleotide does not comprise a detection sequence and, as a consequence, the sample is not contacted, during the method, with a detectable oligonucleotide probe, in particular fluorescently detectable oligonucleotide probe, capable of hybridizing to the first or second oligonucleotide which does not comprise a detection sequence. In such embodiments, one of the third and fourth oligonucleotide may be identical to the first or second oligonucleotide which does not comprise a detection sequence. In such cases, distinction between the two oligonucleotide pairs within the sample, and templates and amplicons derived therefrom, is made through the single detection sequence present on only one of the oligonucleotides of the oligonucleotide pairs.

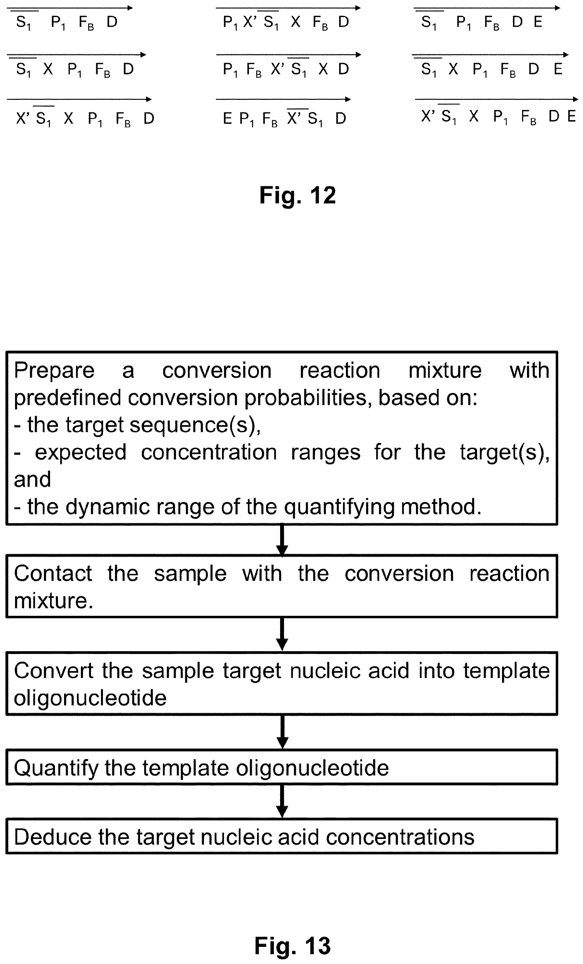

Illustrative examples of oligonucleotides according to the invention are shown in Fig. 12. All the examples are designed to bind of the same target sequence S, but the number and the order of the other sequences on the oligonucleotide can be very different. In these examples, the sequence E stands for a sequence that is not complementary to the other oligonucleotide of the pair and to the target but can play other role, such as providing a secondary structure to the oligonucleotide or controlling its extension conditions.

Exemplary designs of oligonucleotides are illustrated in Fig. 9, where S₁ & S₂ are target sequences, P₁ and P₂ are arbitrary priming sequences, P*z is an arbitrary sequence similar to P₂ but not able to prime the extension, F_{B} & F_{R} are arbitrary sequences for TaqMan^{®} probes, X & Y are arbitrary spacer sequences that are mismatching with the target, D is a hybridization sequence with a Tm lower than S₁ & S₂.

In all appended figures, an arrow reflects a 5"->3' orientation, from the tail of the arrow to the arrowhead. A dash overmounting a letter such as Y̅ reflects the complement of a sequence, in the presence example the complement of sequence Y.

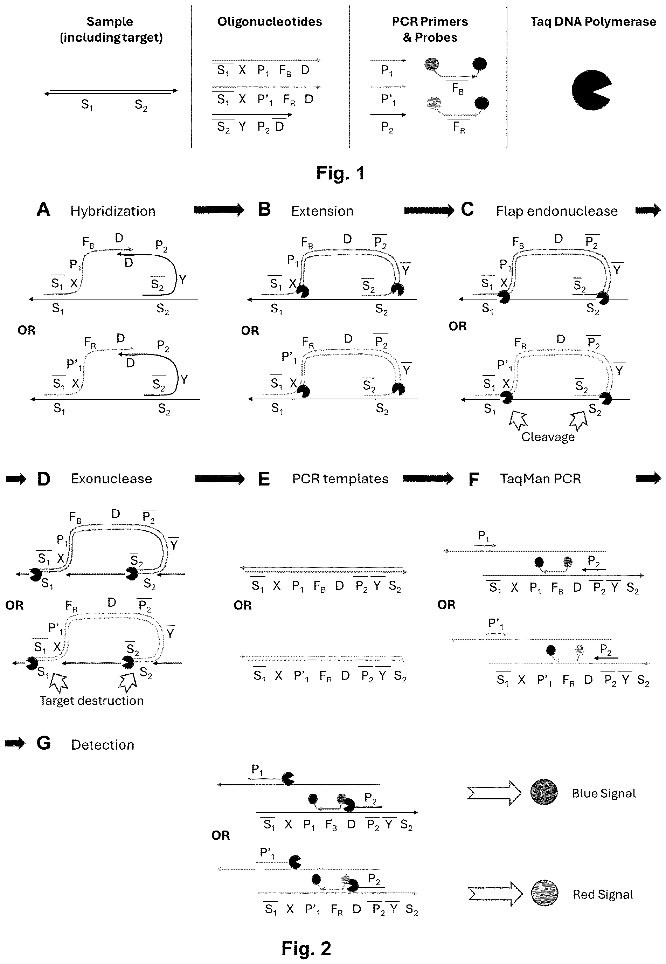

Exemplary designs of oligonucleotides are also illustrated in Fig. 1, wherein S₁ & S₂ are target sequences, P₁, P'₁, and P₂ are arbitrary priming sequences, F_{B} & F_{R} are arbitrary sequences for TaqMan^{®} probes, X & Y are arbitrary sequences that are mismatching with the target, D is a hybridization sequence with a Tm lower than S₁ & S₂. The reagents also comprise primers P₁, P'₁, and P₂ for priming amplification reactions and a Taq polymerase.

An exemplary implementation of the method of the invention, using the oligonucleotides and reagents of Fig. 1 is illustrated in Fig. 2. This example corresponds to an implementation with 3 different oligonucleotides, two oligonucleotides binding to a target sequence S₁ and one oligonucleotide binding to a target sequence S₂. The example may also be viewed as an embodiment in which two oligonucleotide pairs are used which have one oligonucleotide in common, binding to the second target sequence S₂. The common oligonucleotide is shown once in Figure 1, reflecting the presence of 3 different oligonucleotides in the reaction mixture. The embodiment illustrated in Fig. 2 leads to the detection of a blue signal or a red signal, depending on which oligonucleotide pair is elongated, amplified and detected in the method. In the example of Fig. 2, a target nucleic acid is contacted with 3 oligonucleotides and a Taq polymerase. One is specific to the target sequence S₂ and the two others are specific of the target sequence S₁. Out of these two last oligonucleotides, one is designed to be detected with a "blue" signal and the second with a "red" signal. After denaturation of the target by a thermal treatment, while targets and oligonucleotides are in contact and the temperature lowered, two combinations are possible (see A) and in all cases the DNA polymerase elongates the hydridized oligonucleotides starting from the sequence D (see Fig. 2 B). In all the cases (see C), the Taq polymerase reaches a flap and pursues the DNA synthesis while cutting the target in pieces (cleavage). The cut after S2 cleaves the target nucleic acid at a position which leaves sequences S1 and S2, and the regions therebetween intact, making possible another conversion of the target nucleic acid. However, the cut between the sequences S₁ and S₂ makes the target nucleic acid unable to produce another conversion. The target is furthermore destroyed by the exonuclease activity of the Taq polymerase until complete destruction of the sequences S₁ and S₂ (see D). The elongated oligonucleotides (see E) are thermocycled in presence of primers in a standard TaqMan^{®} PCR (see F). The TaqMan^{®} PCR reveals by producing a "blue" or "red" signal, for each target which of the oligonucleotide was bound to the S, sequence (see G).

Alternative exemplary designs of oligonucleotides are illustrated in Fig. 6, where S₁ & S₂ are target sequences, P₁ and P₂ are arbitrary priming sequences, P*₂ is an arbitrary sequence similar to P₂ but not able to prime the extension, F_{B} & F_{R} are arbitrary sequences for TaqMan^{®} probes, X & Y are arbitrary sequences that are mismatching with the target, D is a hybridization sequence with a Tm lower than S₁ & S₂. The reagents also comprise primers P₁, and P₂ for priming amplification reactions and a Taq polymerase.

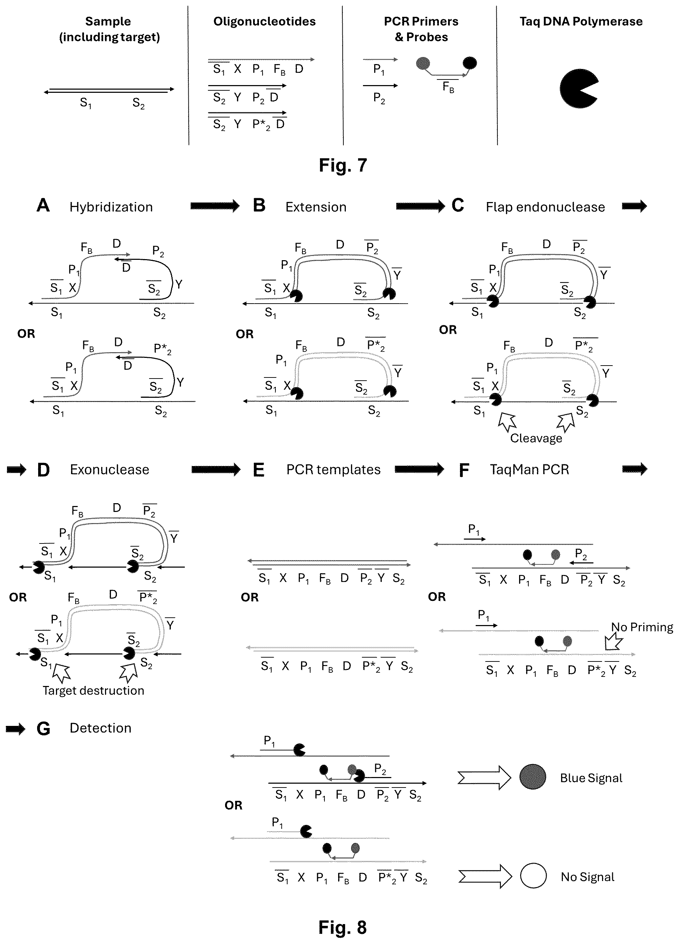

An alternative exemplary implementation of the method of the invention, using the oligonucleotides and reagents of Fig. 7, is illustrated in Fig. 8. In this example, a first oligonucleotide pair is used in conjunction with a "sterile" oligonucleotide comprising a priming sequence which is not amplified during the performance of the method. This example corresponds to an implementation with 3 different oligonucleotides, one oligonucleotide pair capable of being amplified, and a "sterile" oligonucleotide. The example may also be viewed as an embodiment in which two oligonucleotide pairs are used, wherein the second oligonucleotide pair is "sterile", and one of the oligonucleotides of the "sterile" oligonucleotide pair is identical to one oligonucleotide of the first oligonucleotide pair such that three different oligonucleotides are introduced in the sample. The embodiment illustrated in Fig. 8 leads to the detection of a blue signal or no signal, depending on which oligonucleotide pair is elongated, and whether the elongated oligonucleotide is "sterile" i.e., whether it comprises a pair of priming sequences amplified during the performance of the method. In the example of the Fig. 8, a target nucleic acid is contacted with 3 oligonucleotides (i.e. 2 oligonucleotide pairs with a common oligonucleotide) and a Taq polymerase. One is specific to the target sequence S₁ and the signal "blue" and the two others are specific of the target sequence S₂. Out of these two last oligonucleotides, only one can be amplified. After denaturation of the target by a thermal treatment, while targets and oligonucleotides are in contact and the temperature lowered, two combinations are possible (see A) and in all cases the DNA polymerase elongates the hydridized oligonucleotides starting from the sequence D (see B). In all the cases (see C), the Taq polymerase reaches a flap and pursues the DNA synthesis while cutting the target in pieces. The cut between the sequences S₁ and S₂ makes the target nucleic acid unable to produce another conversion. The target is furthermore destroyed by the exonuclease activity of the Taq polymerase until complete destruction of the sequences S₁ and S₂ (see D). The elongated oligonucleotides (see E) are thermocycled in presence of primers in a standard TaqMan^{®} PCR (see F). Nevertheless, only the elongated oligonucleotides that have 2 priming sites (P₁ and P₂) are amplified and produce a detectable signal (blue) (see G). All the other elongated oligonucleotides are not amplified and not detected.

In some embodiments, the method is carried out simultaneously in at least 100 partitions of the sample, preferably at least 1,000 partitions of the sample, still preferably at least 10,000 partitions of the sample, most preferably at least 20,000 partitions in the sample. In some embodiments, the sample may be partitioned between steps (a) and (b), most preferably after mixing all reagents required for the reaction. In some embodiments, the sample may be partitioned between steps (c) and (d), namely after conversion and before amplification.

Partitioning is at the basis of digital PCR (dPCR) reactions. The present method may therefore be carried out as a dPCR reaction. In particular, the partitions may be discrete sites on a chip or droplets in a microfluidics system, such as water-in-oil droplets. Alternatively, the partitions may be wells in a reaction plate comprising wells. The use of a chip or a microfluidics system with portioned droplets is preferred as it provides a higher number of partitions, up to more than 10,000 partitions in current dPCR systems.

In some embodiments, the method comprises predefining at least one probability of conversion reflecting the probability of converting the target nucleic acid into an oligonucleotide elongated from one pair of oligonucleotides, e.g. the first and/or second oligonucleotide and/or the probability of converting the target nucleic acid into an oligonucleotide elongated from another pair of oligonucleotides, e.g. the third and/or fourth oligonucleotides. The probability of conversion corresponds, for instance, to a ratio of the expected probability of converting the target nucleic acid into an oligonucleotide elongated from one pair of oligonucleotides, e.g. the first and/or second oligonucleotide in comparison to the expected probability of converting the target nucleic acid into an oligonucleotide elongated from another pair of oligonucleotides, e.g. the third and/or fourth oligonucleotides. Said probability may be defined, for instance, based on expected concentrations of target nucleic acids within a sample, e.g. where two target nucleic acids are present in different concentrations such as a cell-free tumoral nucleic acid and a cell-free healthy nucleic acid. Said probability may also be defined such as to extend a range of detection of a given target nucleic acid.

In particular, the method comprises applying concentrations of oligonucleotides, e.g. first, second, third and/or fourth oligonucleotides, such that the chances of converting the target nucleic acid into an oligonucleotide elongated from one pair of oligonucleotides, e.g. the first and/or second oligonucleotide, over the chances of converting the target nucleic acid into an oligonucleotide elongated from another pair of oligonucleotides, e.g. the third and/or fourth oligonucleotides correspond approximately to the predefined probability of conversion.

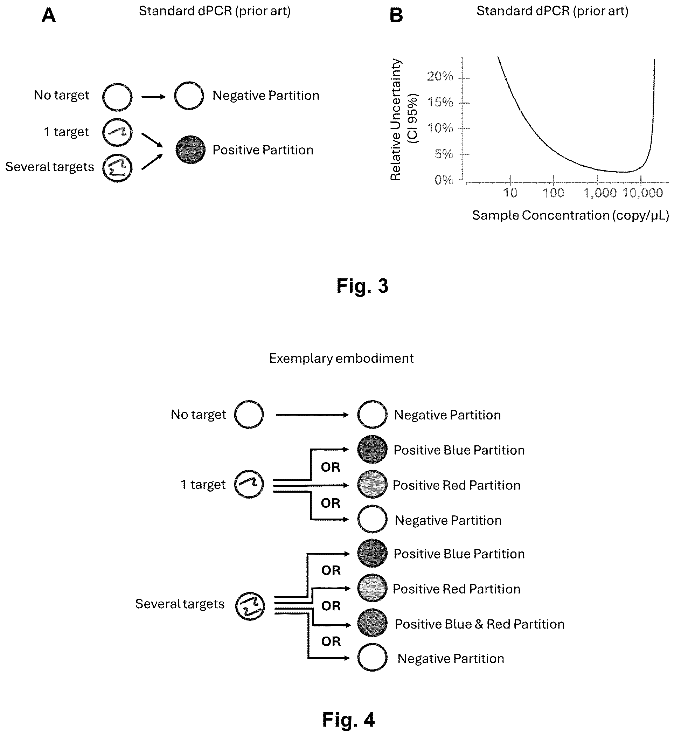

In certain embodiment, said probability is of at least about 2:1, 3:1, 4:1, 5:1, 6:1, 7:1 or 8:1. In some embodiments, said probability is of at least about 9:1, 10:1, 19:1, 29:1, 39:1, 49:1, 59:1, 69:1, 79:1 or 89:1. In some embodiments, said probability is of at least about 99:1 ,or at least 999:1, or at least 9999:1, or at least 99999:1, or at least 999999:1, or at least 9999999:1. A probability of 99:1 means that the target nucleic acid has 99 more chances of being converted into an oligonucleotide template elongated from the first oligonucleotide pair, than into an oligonucleotide template elongated from the second oligonucleotide pair. For instance, the target nucleic acid has 99/100 chances of being converted into an oligonucleotide template elongated from the first oligonucleotide pair and 1/100 chances of being converted into an oligonucleotide template elongated from the second oligonucleotide pair. Tuning the probabilities of conversion in an unequal way allows, for instance, to extend the range of detection of a given target nucleic acid. The oligonucleotide pair with the lower chances of conversion will be dedicated to the detection of higher ranges of concentration of nucleic acid target whereas the oligonucleotide pair with the higher chances of conversion will be dedicated to the detection of lower ranges of concentration of nucleic acid target. A probability of about 99:1, i.e. two logs of difference, is particularly useful to extend the range of quantification of a target nucleic acid since current dPCR methods generally quantify their range over two logs. An appropriate tuning of the probability of detection of each pair of oligonucleotides, e.g. by adjustment of their concentration within the sample, allows extending the range of quantification of a target nucleic acid, at a given confidence interval and given relative uncertainty, e.g. 95% and 5% respectively, over multiple logs of detection, e.g. at least 2, 3, 4, 5, 6, 7 or 8 logs of detection. Commercially available dPCR methods usually have about 2 logs of quantification (relative uncertainty < 5%) and about 4 logs between the limit of detection and the saturation as illustrated in Fig. 3B.

Probability tuning according to an embodiment of the invention is illustrated in Fig. 5, in an embodiment in which one target nucleic acid is detected in the sample. In such illustrative example, the probability is tuned such that the probability of detecting a blue signal is 99 times lower than the probability of detecting a red signal. The blue signal thus extends the quantification limit of detection towards higher ranges of concentration. The illustration ratios allow extending the range of quantification by 2 logs of concentration. Probability tuning is illustrated in Fig. 10 in an embodiment with two different target nucleic acids detected in the sample, wherein the "blue" target is expected to be found at concentration 1000 times higher in the sample than the "red" target. In such illustrative example, the probability is tuned such that the probability of detecting a blue signal in presence of the "blue" target is 1000 times lower than the probability of detecting a red signal in presence of the "red" target. This allows detecting the "blue" target and the "red" target simultaneously in a same sample, in spite of their concentration difference without having to reperform the dPCR assay twice with different sample dilutions.

In certain embodiments, said probability is of about 1:1. A probability of about 1:1 means that the target nucleic acid has about equal chances of being converted into an oligonucleotide template elongated from the first oligonucleotide pair, versus being converted into an oligonucleotide template elongated from the second oligonucleotide pair. Tuning the probabilities of conversion in an equal way allows, for instance to improve the precision of the detection and quantification of a given target nucleic acid. Indeed, in a standard prior art dPCR, a given partition of a partitioned sample will give rise to a binary detection, i.e. presence of one or more occurrences of target nucleic acid, giving rise to a signal; and absence of the target nucleic acid, giving rise to an absence of signal (as illustrated in Fig. 3A). In the method of the invention, a given partition of a partitioned sample gives rise to an improved detection, wherein the presence of one occurrence of target nucleic acid gives rise to a distinct signal, which may be one of two distinct signals issued from the detection of one of the two oligonucleotide pairs, or an absence of signal (e.g. blue or red or no signal, as illustrated in Fig. 4); the presence of more than one occurrence of target nucleic acids gives rise one of the two distinct signals, or to a mixture of the two distinct signals issued from the detection of the two oligonucleotide pairs or to an absence of signal(e.g. blue, red or blue and red, or no signal as illustrated in Fig. 4); the absence of target nucleic acid gives rise to an absence of signal. Therefore, the detection of a mixture of two distinct signals reflects the presence of at least two occurrences of target nucleic acids within the sample, or a partition thereof. The provision of additional information regarding the presence of more than one occurrence of target nucleic acid in the sample or partition, increases the precision of the quantification of the target nucleic acid. Where the chances of conversion are tuned to correspond to a probability ratio of 1:1, the optimal precision evolves by a factor of √n, where n is the number of different oligonucleotide pairs detected in the sample, e.g. V2 where 2 oligonucleotide pairs are present in the sample.

In some embodiments, the method further comprises quantifying the target nucleic acid based on whether, at step (e):
(i) an oligonucleotide elongated from one of the first, second, third and/or fourth oligonucleotides, or amplicons amplified therefrom, is detected; or
(ii) no signal is detected.
Preferably, the detection at step (e) is carried out in each partition of the sample. If one or several sterile oligonucleotides is used in the method, e.g. as third and/or fourth oligonucleotide, the nondetection of a signal in a partition of the sample may be reflective of the absence of the target nucleic acid, or of the presence of one or several occurrences of the target nucleic acid which have been converted into an non-amplified oligonucleotide template using a sterile oligonucleotide and, accordingly, which have not been amplified and detected according to the method. The sum of information originating from detection of the signal or absence of signal from each partition of a partitioned sample allows quantifying the target nucleic acid based on probabilistic distribution rules (Poisson law), taking into account the adjusted probability of converting an oligonucleotide from a fertile or a sterile oligonucleotide pair.

The method may therefore further comprise quantifying the target nucleic acid based on whether, at step (e):
(i) an oligonucleotide elongated from the first and/or second oligonucleotide, or amplicons amplified therefrom, is detected;
(ii) an oligonucleotide elongated from the third and/or fourth oligonucleotide, or amplicons amplified therefrom, is detected;
(iii) oligonucleotide elongated from the first and/or second oligonucleotides, or amplicons amplified therefrom, is detected and an oligonucleotide elongated from the third and/or fourth oligonucleotides, or amplicons amplified therefrom, are detected; or
(iv) no signal is detected.

In particular, the detected signal or absence of signal is indicative of the number of occurrences of the target nucleic acid within the sample or a partition thereof.

In some embodiments, the method comprises applying concentrations of first, second, third and/or fourth oligonucleotides, or any further oligonucleotide such that the chances of converting the target nucleic acid into an oligonucleotide elongated from the first and/or second oligonucleotide, over the chances of converting the target nucleic acid into an oligonucleotide elongated from the third and/or fourth oligonucleotides correspond approximately to the predefined probability of conversion,

In some aspects more than two pairs of oligonucleotides can be used, for instance at least 3, 4, 5, 6, 7, 8, 9 or 10 pairs of oligonucleotides, binding to the same target and/or, as described hereafter, to different targets. An appropriate tuning of the probability of detection of each pair of oligonucleotides, e.g. by adjustment of their concentration within the sample, allows extending the range of quantification of a target nucleic acid, at a given confidence interval and given relative uncertainty, e.g. 95% and 5% respectively, over multiple logs of detection, e.g. at least 2, 3, 4, 5, 6, 7 or 8 logs of detection. The limits of the system, should there be any, correspond to the number of detection channels, e.g., fluorescent channels or combination of them, in the apparatus used for detection of the signals corresponding to each oligonucleotide pair.

In some aspects, the invention relates to a method for detecting and/or quantifying at least two different nucleic acid targets in a sample, wherein said method comprises distinctly detecting and/or quantifying each of the nucleic acid targets into oligonucleotides according to the method described herein. The quantification of each nucleic acid may use at least one pair of oligonucleotides as defined herein, wherein said pair of oligonucleotides is specific for one of the target nucleic acid targets in the sample. Hence, the method of the invention finds application in multiplex PCR, for detecting and quantifying several targets in a single reaction sequence.

Each aspect of the method relating to the detection and/or quantification of one target nucleic acid is applicable to the detection and/or quantification of several target nucleic acids. Hence, for each target nucleic acid, two or more sets of specific oligonucleotide pairs can be used, in particular including a "sterile" pair of oligonucleotide pairs not amplified during the method, which serve to tune the probability of detection of a target nucleic acid in the sample. This allows, for instance, to detect, simultaneously in the same sample or partition, different target nucleic acids present in different concentrations, particularly in very different concentrations, e.g. with an at least 2 log difference, in particular at least 3 log or at least 4 log , 5 log, 6 log, 7 log or 8 log or more differences of concentration. This is particularly useful in applications where an important difference of concentration exists between two nucleic acids of interest, e.g., to quantify cell-free tumoral nucleic acid, in minor quantities in comparison to cell-free non-tumoral nucleic acid. For the same reasons, such embodiments are useful to quantify cell-free fetal nucleic acid.

Various additional variations may also be useful in the method of the invention.

For instance, the sample may be contacted with reagents, preferably all reagents, required for amplification and detection of the oligonucleotides at step (a). In some embodiments, the method does not involve further addition of reagents after step (a). This "one-pot reaction" embodiment is particularly useful when the sample is partitioned between step (a) and (b) such that all reagents are added before partitioning.

In some embodiments, said oligonucleotides of the first pair of oligonucleotides and, optionally, second pair of oligonucleotides are synthetic oligonucleotides.

In some embodiments, said oligonucleotides of the first pair of oligonucleotides and, optionally, second pair of oligonucleotides comprise a cleavage sequence. The cleavage sequence may be a recognition site of an endonuclease, e.g. a restriction enzyme. In some embodiments, the cleavage sequence serves to eliminate any remaining oligonucleotide within the device used for detection, i.e. well, tube, chip etc..., before performing another cycle of sample conversion, detection and quantification. In one embodiment, the method thus comprises removing the sample from the reaction compartment in which the method occurs and contacting the reaction compartment with at least one restriction enzyme to cleave remaining oligonucleotides. In another embodiment, the method thus comprises contacting the sample with a restriction enzyme before any amplification in order to remove any possible contamination arising from previous samples. In a preferred embodiment, all pairs of oligonucleotides from a given assays have the same cleavage sequence.

Exemplary steps of the method of the invention are shown in Fig. 13. As illustrated, the method may comprise:
- A step of preparing a conversion reaction mixture with predefined conversion probabilities, based on:
   ∘ the target sequence(s)
   ∘ expected concentration ranges for the target(s), and
   ∘ the dynamic range of the quantifying method;
- a step of contacting the sample with the conversion reaction mixture;
- a step of converting the sample target nucleic acid into a template oligonucleotide;
- a step of quantifying the template nucleic acid;
- a step of deducing the target nucleic acid concentration.

In some aspects, the invention relates to a set of oligonucleotides for use in a method for converting and optionally detecting and/or quantifying a target nucleic acid, in particular the method of any one of the aspects and embodiments described in the present specification, wherein said set of oligonucleotides comprises a first pair of oligonucleotide, comprising:
(i) a first oligonucleotide comprising a first target-specific sequence and a first bridging sequence, wherein the first target-specific sequence is complementary to a first target region of the target nucleic acid; and
(ii) a second oligonucleotide comprising a second target-specific sequence and a second bridging sequence, preferably in the 3'-position relative to the second target-specific sequence, wherein the second target-specific sequence is complementary to a second target region of the target nucleic acid and the second bridging sequence is complementary to the first bridging sequence.

Preferably, the first bridging sequence is in the 3'-position relative to the first target-specific sequence, and/or the second bridging sequence is in the 3'-position relative to the second target specific sequence.

In some embodiments, the set of oligonucleotides further comprises at least one second pair of oligonucleotides.

In some embodiments, said second pair of oligonucleotides is specific for a second target nucleic acid, different from the target nucleic acid targeted by the first pair of oligonucleotides. By "different", it is meant that the target nucleic acids differ in their nucleotide sequence. In such embodiment, the second pair of oligonucleotides may have the same design and/or features as the first pair of oligonucleotides, with different sequences, e.g. different target-specific sequences, different bridging sequences, different priming sequences and/or detection sequences than the first oligonucleotide pair, as described in relation to the method of the invention. In particular, the second oligonucleotide pair specific for a second target nucleic acid may comprise a third oligonucleotide and a fourth oligonucleotide, wherein:
- the third oligonucleotide comprises a third target-specific sequence and a third bridging sequence, preferably located in the 3'-position relative to the third target-specific sequence, wherein the third target-specific sequence is complementary to a first target region of the second target nucleic acid, and
- the fourth oligonucleotide comprises a fourth target-specific sequence and a fourth bridging sequence, preferably located in the 3'-position relative to the fourth target-specific sequence, wherein the fourth target-specific sequence is complementary to a second target region of the second target nucleic acid and the fourth bridging sequence is complementary to the third bridging sequence.

In other embodiments, said second pair of oligonucleotides is specific for the target nucleic acid, i.e. the same target nucleic acid as the first pair of oligonucleotides, and wherein said second pair of oligonucleotides comprises a third oligonucleotide and a fourth oligonucleotide, wherein:
- the third oligonucleotide comprises a third target-specific sequence and a third bridging sequence, preferably located in the 3'-position relative to the third target-specific sequence, wherein the third target-specific sequence is complementary to the first target region of the target nucleic acid such as to compete with the first target-specific sequence for hybridization to the target nucleic acid, and
- the fourth oligonucleotide comprises a fourth target-specific sequence and a fourth bridging sequence, preferably located in the 3'-position relative to the fourth target-specific sequence, wherein the fourth target-specific sequence is complementary to the second target region of the target nucleic acid such as to compete with the first target-specific sequence for hybridization to the target nucleic acid,
wherein the fourth bridging sequence is complementary to the third bridging sequence. Preferably, the fourth bridging sequence is complementary to the first bridging sequence and/or the third bridging sequence is complementary to the second bridging sequences,

In some embodiments, one of the third or oligonucleotides is identical to one the first and second oligonucleotides, such that the set of oligonucleotides comprises three different oligonucleotides. In other words, the first and second pair of oligonucleotides may have one oligonucleotide in common.

All aspects and embodiments described in connection with the methods described previously applies to the set of oligonucleotides as described herein.

In some aspects, the invention may relate to a kit for use in a method for detecting a target nucleic acid, comprising:
(i) a set of oligonucleotides as described in the present specification;
and one or more of:
(ii) one or more pair of primers for hybridization to the oligonucleotides of an oligonucleotide pair of the set of oligonucleotides, or to a complement thereof; and
(iii) one or more oligonucleotide probes, wherein each probe is suitable for detecting an oligonucleotide of the set of oligonucleotides.

All aspects and embodiments of the first and/or second oligonucleotides described in connection with the methods described previously applies to the set of oligonucleotides as described herein.

In particular, said primers and oligonucleotide probes may be as described in the present specification. In particular, each primer of the primer pair is suitable for hybridization to an oligonucleotide of the set of oligonucleotides, or to a complement thereof. In particular, the primer pair may comprise a first primer comprising a sequence complementary to a first priming sequence within the first oligonucleotide, or a complement thereof and a second primer comprising a sequence complementary to a second priming sequence within the second oligonucleotide, or a complement thereof. In particular, the primers are suitable to amplify, preferably by PCR, an oligonucleotide elongated from the first oligonucleotide pair or from any further oligonucleotide pair. In particular, the kit may comprise two primer pairs, suitable for hybridization, respectively, to the first oligonucleotide pair or complements of said oligonucleotides, and the second oligonucleotide pair or complements of said oligonucleotides. In particular, the kit may comprise two primer pairs respectively suitable to amplify, preferably by PCR, a template oligonucleotide elongated from the first oligonucleotide pair and a template oligonucleotide elongated from the second oligonucleotide pair, respectively. In some embodiments, one primer of the second primer pair is identical to one primer of the first primer pair, such that the first and second primer pairs of the kit together comprise 3 different primers. In other terms, the kit comprises one primer pair and an auxiliary primer, as described in the present specification in relation to the method of the invention.

In some embodiments, the oligonucleotide probes may be detectable oligonucleotide probes, such as fluorescently detectable oligonucleotide probes. TaqMan^{®} probes can be used, for instance.

In some embodiment, the kit further comprises at least one of:
(i) a nucleic acid polymerase;
(ii) reagents and buffers for PCR amplification reaction;
(iii) a notice of instructions or specification.

The notice of instructions or specification may comprise or consists in a document specifying the sequence of the target nucleic acid, and/or the sequences of the first and/or second target regions within the target nucleic acid. Said document may be in the form of a paper notice, or in any other printed form and/or an electronic form and may include, for instance, portable document format (pdf) documents, documents editable by a text processor, emails, instant messaging documents etc.., without any limitation on the type and form of the document.

In some embodiments, the reagents and buffers for PCR amplification comprise any one of magnesium chloride, potassium chloride, dNTP, pH buffer (e.g., tris(hydroxymethyl)aminomethane) or additives such as EDTA, Dimethyl sulfoxide (DMSO), glycerol, formamide, bovine serum albumin (BSA), ammonium sulfate, polyethylene glycol (PEG), gelatin, nonionic detergents (e.g., Tween 20, Triton X-100), N,N,N-trimethylglycine (betaine) and their mixtures.

In a further aspect, the present invention relates to the use of a set of oligonucleotides as described herein, or a kit as described herein in the conversion, detection and/or quantification of a target nucleic acid, preferably by digital PCR.

The methods, oligonucleotides and kits of the present invention be used in various fields of application, such as in diagnosis or in epidemiology.

Diagnostic industry may use it when the concentration of the target needs to be known precisely but the concentration level is unknown ex ante, for example, for HIV RNA quantification in blood tracking (7 orders of magnitude range).

Diagnostic industry can also use it when several target simultaneously that are present at different concentrations. For example, the proportion of tumoral cell-free DNA in blood is used in cancer tracking. Both healthy cells and tumoral cells release cfDNA but the DNA coming from the second is several orders of magnitude smaller than the first, quantifying both precisely and simultaneously is needed.

In epidemiology, the same idea of quantifying simultaneously the main signal and the interesting traces can be used to track simultaneously the main variant of a pathogen and the emerging variants in wastewater.

### REFERENCES

Throughout this application, various references describe the state of the art to which this invention pertains. The disclosures of these references are hereby incorporated by reference into the present disclosure.

## Claims

1. A method for synthesizing a template oligonucleotide from a nucleic acid target in a sample, and optionally for detecting and/or quantifying said nucleic acid target, wherein said method comprises:
a) contacting a sample which contains a target nucleic acid with a nucleic acid polymerase and a first pair of oligonucleotides comprising a first and a second oligonucleotides, wherein:
(i) the first oligonucleotide comprises a first target-specific sequence and a first bridging sequence, preferably in the 3'-position of the first target-specific sequence, wherein the first target-specific sequence is complementary to a first target region of the target nucleic acid; and
(ii) the second oligonucleotide comprises a second target-specific sequence and a second bridging sequence, preferably in the 3'-position of the second target-specific sequence, wherein the second target-specific sequence is complementary to a second target region of the target nucleic acid, and wherein the second bridging sequence is complementary to the first bridging sequence,
b) hybridizing the first oligonucleotide to the first target region of the target nucleic acid and hybridizing the second oligonucleotide to the second target region of the target nucleic acid, whereby the first and second bridging sequences hybridize with each other;
c) elongating, by the nucleic acid polymerase, the first and/or second oligonucleotide hybridized by a bridging sequence, thereby inducing the degradation of the target nucleic acid sequence, thus converting the target nucleic acid into the elongated oligonucleotide, providing a template oligonucleotide; and, optionally
d) amplifying the elongated oligonucleotide, preferably by PCR amplification;
e) detecting the presence in the sample of the elongated oligonucleotide or an amplicon amplified therefrom.

2. The method of claim 1, wherein the target nucleic acid is degraded at step (c) by hydrolysis of the target nucleic acid catalyzed by a 5'-flap endonuclease activity and a 5'->3' exonuclease activity, preferably wherein the nucleic acid polymerase has 5'-flap endonuclease activity and 5'->3' exonuclease activity, and the target nucleic acid sequence is degraded at step (c) by the 5'-flap endonuclease activity and 5'->3' exonuclease activity of the nucleic acid polymerase.

3. The method of claim 1 or 2, wherein the sample is contacted, at step (a), with at least one second pair of oligonucleotides comprising a third oligonucleotide and a fourth oligonucleotide, wherein:
(iii) the third oligonucleotide comprises a third target-specific sequence and a third bridging sequence, preferably located in the 3'-position of the third target-specific sequence, wherein the third target-specific sequence is complementary to the first target region of the target nucleic acid; and
(iv) the fourth oligonucleotide comprises a fourth target-specific sequence and a fourth bridging sequence, preferably located in the 3'-position of the fourth target-specific sequence, wherein the fourth target-specific sequence is complementary to the second target region of the target nucleic acid, wherein the fourth bridging sequence is complementary to the third bridging sequence,
wherein the fourth bridging sequence is complementary to the first bridging sequence and/or the third bridging sequence is complementary to the second bridging sequence,
wherein at step (b), the third oligonucleotide competes with the first oligonucleotide for hybridization to the target nucleic acid and the fourth oligonucleotide competes with the second oligonucleotide for hybridization to the target nucleic acid, wherein:
- if the target nucleic acid is hybridized to the first and second oligonucleotides, step (c) comprises elongating, by the nucleic acid polymerase, the first and/or second oligonucleotides hybridized by a bridging sequence, thereby inducing the degradation of the target nucleic acid sequence, whereby the target nucleic acid is converted into an oligonucleotide elongated from the first and/or second oligonucleotides; or
- if the target nucleic acid is hybridized to the third and fourth oligonucleotides, step (c) comprises elongating, by the nucleic acid polymerase, the third and/or fourth oligonucleotides hybridized by a bridging sequence, thereby inducing the degradation of the target nucleic acid sequence, whereby the target nucleic acid is converted into an oligonucleotide elongated from the third and/or fourth oligonucleotides.

4. The method of claim 3, wherein step (d) comprises exponentially amplifying an elongated oligonucleotide synthesized from the first pair of oligonucleotides and does not comprise exponentially amplifying an elongated oligonucleotide synthesized from said second pair of oligonucleotides,
preferably wherein the first and second oligonucleotides comprise priming sequences, wherein step (d) comprises contacting the sample with a primer pair hybridizing to said priming sequences or their complement, for exponentially amplifying an elongated oligonucleotide synthesized from the first pair of oligonucleotides, wherein the method does not comprise contacting the sample with a primer pair for amplifying an elongated oligonucleotide synthesized from the second pair of oligonucleotides.

5. The method of claim 3 or 4, wherein the third oligonucleotide is identical to the first oligonucleotide, or wherein the fourth oligonucleotide is identical to the second oligonucleotide.

6. The method of any one of claims 1 to 5, wherein at step (e), each elongated oligonucleotide is detected by a distinctly detectable oligonucleotide probe or is not detected.

7. The method of any one of claims 1 to 6, wherein the sample is partitioned and the method is carried out simultaneously in more than one partition of the sample, preferably at least 100 partitions of the sample, preferably wherein the sample is partitioned between steps (a) and (b).

8. The method of claim 7, wherein the method further comprises quantifying the target nucleic acid based on whether, at step (e):
(i) an oligonucleotide elongated from one of the first, second, third and/or fourth oligonucleotides, or amplicons amplified therefrom, is detected; or
(ii) no signal is detected,
preferably wherein the detection at step (e) is carried out in each partition of the sample.

9. The method of claim 7, wherein the method comprises quantifying the target nucleic acid based on whether, at step (e):
(i) an oligonucleotide elongated from the first and/or second oligonucleotides, or amplicons amplified therefrom, is detected;
(ii) an oligonucleotide elongated from the third and/or fourth oligonucleotides, or amplicons amplified therefrom, is detected;
(iii) an oligonucleotide elongated from the first and/or second oligonucleotides, or amplicons amplified therefrom, is detected and an oligonucleotide elongated from the third and/or fourth oligonucleotides, or amplicons amplified therefrom, is detected; or
(iv) no signal is detected;
in particular wherein the detected signal or absence of signal is indicative of the number of occurrences of the target nucleic acid within the sample or a partition thereof.

10. The method of any one of claims 1 to 9, comprising predefining at least one probability of conversion reflecting the probability of converting the target nucleic acid into an oligonucleotide elongated from the first and/or second oligonucleotide in comparison to the probability of converting the target nucleic acid into an oligonucleotide elongated from the third and/or fourth oligonucleotides, preferably wherein the method comprises applying concentrations of first, second, third and/or fourth oligonucleotides; such that the chances of converting the target nucleic acid into an oligonucleotide elongated from the first and/or second oligonucleotide, over the chances of converting the target nucleic acid into an oligonucleotide elongated from the third and/or fourth oligonucleotides correspond approximately to the predefined probability of conversion, for instance wherein said probability is of at least about 9:1, in particular of at least 99:1, or wherein said probability is of about 1:1.

11. A method for detecting and/or quantifying at least two different nucleic acid targets in a sample, wherein said method comprises synthesizing oligonucleotide templates from each nucleic acid target, and optionally amplifying said oligonucleotide templates and detecting the amplicons thereof, according to the method of any one of claims 1 to 10.

12. A set of oligonucleotides for use in a method for converting and optionally detecting and/or quantifying a target nucleic acid, in particular the method of any one of claims 1 to 11, comprising a first pair of oligonucleotides, comprising:
(i) a first oligonucleotide comprising a first target-specific sequence and a first bridging sequence, preferably in the 3'-position of the first target-specific sequence, wherein the first target-specific sequence is complementary to a first target region of the target nucleic acid; and
(ii) a second oligonucleotide comprising a second target-specific sequence and a second bridging sequence, preferably in the 3'-position of the second target-specific sequence, wherein the second target-specific sequence is complementary to a second target region of the target nucleic acid and the second bridging sequence is complementary to the first bridging sequence.

13. The set of oligonucleotides of claim 12, further comprising at least one second pair of oligonucleotides, wherein said second pair of oligonucleotides is specific for a second target nucleic acid, or wherein said second pair of oligonucleotides is specific for the same target nucleic acid as the first pair of oligonucleotides, and wherein said second pair of oligonucleotides comprises a third oligonucleotide and a fourth oligonucleotide, wherein:
(iii) the third oligonucleotide comprises a third target-specific sequence and a third bridging sequence, preferably located in the 3'-position of the third target-specific sequence, wherein the third target-specific sequence is complementary to the first target region of the target nucleic acid such as to compete with the first target-specific sequence for hybridization to the target nucleic acid;
(iv) the fourth oligonucleotide comprises a fourth target-specific sequence and a fourth bridging sequence, preferably located in the 3'-position of the fourth target-specific sequence, wherein the fourth target-specific sequence is complementary to the second target region of the target nucleic acid such as to compete with the second target-specific sequence for hybridization to the target nucleic acid, wherein the fourth bridging sequence is complementary to the third bridging sequence, and
wherein the fourth bridging sequence is complementary to the first bridging sequence and/or the third bridging sequence is complementary to the second bridging sequences.

14. A kit for use in a method for detecting a target nucleic acid, comprising:
(i) a set of oligonucleotides according to claim 12 or 13;
and one or more of:
(ii) one or more pair of primers for hybridization to the oligonucleotides of an oligonucleotide pair of the set of oligonucleotides, or to a complement thereof; and
(iii) one or more oligonucleotide probes, wherein each probe is suitable for detecting an oligonucleotide of the set of oligonucleotides;
wherein the kit optionally further comprises at least one of:
(iv) a nucleic acid polymerase;
(v) reagents and buffers for PCR amplification reaction; and
(vi) a notice of instructions or specification.

15. Use of a set of oligonucleotides according to claim 12 or 13, or a kit according to claim 14, in a method for detecting and/or quantifying a target nucleic acid, preferably by digital PCR.
